# EUROPEAN PATENT APPLICATION

(11) **EP 1 122 313 A1**
(43) Date of publication of application: **08.08.2001**
(21) Application number: 99969957.2
(22) Date of filing: 07.10.1999
(51) Int. Cl.: C12N 15/12, C07K 14/705, C07K 16/28, G01N 33/566, G01N 33/15, A61K 45/00, A61K 31/70

(54) **NOVEL G PROTEIN-COUPLED RECEPTOR PROTEIN AND DNA THEREOF**

(30) Priority: 08.10.1998 JP 28660798
(71) Applicant: Takeda Chemical Industries, Ltd., Osaka-shi, Osaka 541-0045 (JP)
(72) Inventor: WATANABE, Takuya, Tsukuba-shi Ibaraki 305-0821 (JP); TERAO, Yasuko, Tsukuba-shi Ibaraki 305-0034 (JP); MATSUI, Hideki, Tsukuba-shi Ibaraki 305-0044 (JP)
(74) Representative: Keller, Günter, Dr.
(86) International application number: JP9905534
(87) International publication number: WO0020580

(57) **Abstract**

The present invention relates to a rat cerebellum-derived G-protein-coupled receptor protein, its partial peptides, and salts thereof, and a nucleic acid and its derivatives that encodes the receptor protein.

The rat cerebellum-derived G-protein-coupled receptor protein of this invention and the nucleic acid and its derivatives encoding the said protein can be used for determination of ligands (agonists) for the G-protein-coupled receptor protein of this invention, prophylactic and/or therapeutic drugs for diseases related to dysfunction of the G-protein-coupled receptor protein of this invention, gene diagnostic drugs, and methods for screening compounds that change the expression level of the receptor protein of this invention and its partial peptides.

## Description

### FIELD OF THE INVENTION

The present investion relates to a novel G-protein-coupled receptor protein derived from the rat cerebellum or salts thereof, and DNA enconding the protein.

### BACKGROUND ART

Physiological active substances such as various hormones and neurotransmitters regulate the biological function via specific receptor proteins present on cell membranes. Many of these receptor proteins are coupled with guanine nucleotide-binding protein (G-protein) and perform intrancellular singnal transduction via activation of G-proptein. These receptor proteins possess a common structure containing seven transmembrane domains. Therefore, these proteins are generically called G-protein-coupled receptor protein or senven transmembrane receptor proteins (7TMR).

G-protein-coupled receptor proteins are present on the biogenic cells or the cell surface of each functional cells of the orans, and play important physiological roles as the targets of molecules that regulate the fuctions of the cells and organs such as hormones, neurotransmitters and physiologically active substances. Receptors transmit signals into cells via binding with physiological active substances, and the signals induce various reactions such as activation and repression of the cells.

Clarification of the relationship between the substances that regulate complex functions in various biogenic cells and the organs, and specific receptor proteins thereof, especially G-protein-coupled receptor proteins, elucidates the functions of various biogenic cells and organs and provides very important means for the pharmaceutical development closely related to the functions.

For example, in various biogenic organs, the regulation of physiological function is controlled via various hormones, hormone-like substances, neurotransmitters, and/or physiological active substances. Especially, physiological active substances are present in various regions in vivo, and regulate the physiological functions via the corresponding receptor proteins. There are many unknown biological hormones, neutrotransmitters, and other physiological active substances, and the structure of many receptor proteins has not been reported. Moreover, the presence of receptor subtypes remains unclear even for many known receptor proteins.

Clarification of the relationship between substances that regulate complex biological functions and its specific receptor proteins is an important means for pharmaceutical development. Furthremore, for efficfient screening of agonists and antagonists of receptor proteins in pharmaceutical development, it was necessary to elucidate the functions of receptor protein genes expressed in vivo and to express the genes in appropriate expression systems.

Recently, random analysis of cDNA sequences has been actively peformed as a method for analyzing genes expressed in vivo, and the obtained cDNA fragment sequences have been registered and published to data bases as Expressed Sequence Tag (EST). However, most EST contains only the sequence information, and the function is difficult to deduce.

Substances that inhibit binding between G-protein-coupled proteins and physiological active substances (i.e. ligands) and substances that bind and induce signal transduction similar to those induced by physiological active subsntaces (i.e. ligands) have been used as pharmaceuticals as antagonists and agonists specific for the receptors that regulate the biological functions. Therefore, discovery of a novel G-protein-coupled receptor protein that can be targeted for pharmaceutical development and cloning of the gene (e.g. cDNA) are very important means in search for specific ligands, agonists, and antagonists of the novel G-protein-coupled receptor protein.

However, not all G-protein-coupled receptors have been discovered. There are also many unknown G-protein-coupled receptors and those for which the corresponding ligands are unidentified, that is orphan receptors. Therefore, search and functional elucidation for a novel G-protein-coupled receptor are awaited.

G-protein-coupled receptors are useful in searching for a novel physiological active substance (i.e. ligand) using the signal transduction activity as the index and in search for agonists and antagonists of the receptor. Even if no physiological ligand is found, agonists and antagnonist of the receptor may be prepared by analyzing the physiological action of the receptor via the receptor inactivation experiment (knockout animal). Ligands, agonists, and antagonists of the receptor are expected to be used as prophylactic/therapeutic and diagnostic drugs for diseases associated with dysfunction of the G-protein-coupled receptor.

Reduction or elevation of functions of the G-protein-coupled receptors due to abberation of the receptor genes causes some disorder in many cases. In such cases, the G-protein-coupled receptor can be used not only for administration of antagonist and agonist of the receptor, but also for gene therapy by introduction of the receptor gene and/or the antisense-nucleic acid of the receptor gene into the body (or the specific organ). In the gene therapy, the base sequence of the receptor gene is essential for investigating deletion and mutation in the gene, and the receptor gene can also be used for prophylactic/therapeutic and diagnostic drugs for diseases related to dysfunction of the receptor.

### DISCLOSURE OF THE INVENTION

The present invention provides a novel G-protein-coupled receptor protein that is useful as described above. The present investion provides a novel G-protein-coupled receptor protein and its partial peptides and its salts, polynucleotides (DNA, RNA, and its derivatives) containing polynucleotides (DNA, RNA, and its derivatives) encoding said G-protein-coupled receptor protein or said partial peptides, recombiant vectors containing said polynucleotides, transformants containing said recominant vectors, methods for manufacturing said G-protein-coupled receptor protein or said salts thereof, antibodies against said G-protein-coupled receptor protein, said partial peptides or said salts thereof, compounds that change the expression level of said G-protein-coupled receptor protein, methods for determining ligands for said G-protein-coupled receptor protein, methods for screening compounds (antagonists and agonists) and salts thereof that change the binding property between ligands and said G-protein-coupled receptor protein, kits for screening, compounds (antagonists and agonists) and its salts that change the binding property between ligands obtained using said screening method or said screening kit and said G-protein-coupled receptor protein, and pharmaceuticals containing the compounds (antagonists and agonists) that change the binding property between ligands and said G-protein-coupled receptor protein, or the compounds or its salts that change the expression level of said G-protein-coupled receptor protein.

The inventors performed extensive studies and succeeded in isolation of cDNA encoding the novel rat cerebellum-derived G-protein-coupled receptor protein and analysis of the entire base sequence of the cDNA based on the EST information prepared by the degenerated PCR method. When the base sequence was translated into the amino acid sequence, the first to the seventh transmembrane domains were observed on the hydrophobicity plot, confirming that the protein encoded by the cDNA is a seven transmembrane G-protein-coupled receptor protein. The inventors further proceeded the study based on these findings, and completed the present invention.

The present invention relates to:
(1) G-protein-coupled receptor protein and its salts characterized by containing amino acid sequences identical or substantially identical to the amino acid sequence presented by SEQ ID:1.
(2) Partial peptides and its salts of the G-protein-coupled receptor protein described in (1).
(3) Polynucleotides containing polynucleotides having the base sequence encoding the G-protein-coupled receptor protein dscribed in (1).
(4) Polynucleotides described in (3), said polypepides are DNA.
(5) Polynucleotides described in (3) having the base sequence presented by SEQ ID:2.
(6) Recombinant vectors containing the polynucleotides described in (3).
(7) Transformants transformed by the recombiant vectors described in (6).
(8) Methods for manufucturing the G-protein-coupled receptor protein or its salts described in (1) characterized by culturing the ransformants described in (7) and by producing the G-protein-coupled receptor protein described in (1).
(9) Antibodies against the G-protein-coupled receptor protein described in (1) and the partial peptides or salts thereof described in (2).
(10) The antibodies described in (9) wherein said antibodies neutralize signal transduction of the G-protein-coupled receptor protein described in (1).
(11) Diagnostic drugs containing the antibodies described in (9).
(12) Ligands for the G-protein-coupled receptor protein and its salts described in (1) wherein said lignds can be obtained using the G-protein-coupled receptor protein described in (1) or the partial peptides ot its salts described in (2).
(13) Pharmaceuticals containing the ligands of the G-protein-coupled receptor protein described in (12).
(14) Methods for determining ligands for the G-protein-coupled receptor protein or the salts thereof described in (1) wherein said method is characterized by using the G-protein-coupled receptor protein described in (1), and the partial peptides or the salts thereof described in (2) .
(15) Methods for screening compounds or its salts that change the binding property between ligands and the G-protein-coupled receptor protein or its salts described in (1) wherein said methods are characterized by using the G-protein-coupled receptor protein described in (1) or the partial peptides, or salts thereof described in (2) .
(16) Kits for screening compounds or its salts that change the binding property between ligands and the G-protein-coupled receptor protein or its salts described in (1) wherein said ligands are characterized by using the G-protein-coupled receptor protein described in (1), the partial peptides or its salts described in (2).
(17) Compounds and its salts that change the binding property between ligands and the G-protein-coupled receptor protein or its salts described in (1) wherein said ligands can be obtained using the screening methods described in (15) or the screening kits described in (16).
(18) Pharmaceuticals containing compounds or its salts that change the binding property between ligands obtained using the screening methods described in (15) or the screening kits described in (16) and the G-protein-coupled receptor protein or its salts described in (1).
(19) Polynucleotides that hybridize to the polynucleoptides described in (3) under a high stringent condition.
(20) Polynucletides containing base sequences complementary to the polynucleotides described in (3) and its partial sequences.
(21) Methods for quqntifying mRNA of the G-protein-coupled receptor protein described in (1) characterized by using the polynucleotides dscribed in (3) or its partial sequences.
(22) Methods for quantifying the G-protein-coupled receptor protein described in (1) characterized by use of the antibodies described in (9).
(23) Methods for diagnosing diseases related to functions of G-protein-coupled receptor protein described in (1) characterized by using the quntification methods described in (21) or (22).
(24) Methods for screening compounds or its salts that change the expression level of the G-protein-coupled receptor protein described in (1) characterized by using the quantification methods descriebd in (21).
(25) Methods for screening compounds or its salts that change the amount of the G-protein-coupled receptor protein described in (1) in cell membranes characterized by using the quantification methods described in (22).
(26) Compounds or its salts that change the expression level of the G-protein-coupled receptor protein described in (1) obtained using the screening methods described in (24).
(27) Compounds or its salts that change the amount of the G-protein-coupled receptor protein described in (1) in cell membranes obtained using the screening methods described in (25).

Furthermore, the present invention provides:
(28) The G-protein-coupled receptor protein or its salts described in (1) comprising: ① amino acid sequence presented by SEQ ID:1, and amino acid sequences presented by SEQ ID:1 in which one, two, or more amino acids (preferably 1 - 30 amino acids, more preferably 1 - 9 amino acids, and most preferably several (1 - 5) amino acids) are deleted; ② amino acid sequences presented by SEQ ID:1 in which one, two, or more amino acids (preferably 1 - 30 amino acids, more preferably 1 - 10 amino acids, and most preferably several (1 - 5) amino acids) are added; ③ amino acid sequences presented by SEQ ID:1 in which one, two, or more amino acids (preferably 1 - 30 amino acids, more preferably 1 - 10 amino acids, and most preferably several (1 - 5) amino acids) are substituted by other amino acids; and ④ combinations of the above amino acid sequences.
(29) Methods for determining ligands described in (14) wherein said methods are characterized by contacting test compounds with the G-protein-coupled receptor protein or the salts thereof described in (1), or partial peptides or the salts thereof described in (2).
(30) The methods for determining the ligand described in (29) in which ligands are, for example, angiotensin, bombesin, canavinoid, cholecystokinin, glutamine, serotonin, melatonin, neuropeptide Y, opioids, purines, vasopressin, oxytocin, PACAP, secretin, glucagon, calcitnonin, adrenomedulin, somatostatin, GHRH, CRF, ACTH, GRP, PTH, vasoactive intestinal and related polypeptide (VIP), somatostatin, dopamine, motilin, amyrin, bradykinin, calcitonin gene-related peptide (CGRP), leukotriens, pancreastatin, prostaglandins, thromboxane, adenosine, adrenaline, α and β-chemokines (e.g. IL-8, GROα, GROβ, GROγ, NAP-2, ENA-78, PF4, IP10, GCP-2, MCP-1, HC14, MCP-3, I-309, MIP1α, MIP-1β, and RANTES), endothelin, enterogastrin, histamine, neurotensin, TRH, pancreatic polypeptide, and galanin.
(31) The method for screening described in (15) in which (i) contact of a ligand to the G-protein-coupled receptor protein or its salts described in (1) or the partial peptides or its salts described in (2) is compared with (ii) contact of the ligand and a test compound to the G-protein-coupled receptor protein or its salts described in (1) or the partial peptides or its salts described in (2).
(32) The method for screening compounds and its salts that change the binding property bewteen ligands and the G-protein-coupled receptor protein or its salts described in (1) in which the amount of a labeled ligand bound to the G-protein-coupled receptor protein or its salts described in (1) or the partial peptides or its salts described in (2) is measured after: (i)contacting the labeled ligand to the G-protein-coupled receptor protein or its salts described in (1)and compared with after (ii) contacting the labeled ligand and a test compound to the G-protein-coupled receptor protein or its salt formds described in (1) or the partial peptides or its salts described in (2).
(33) The method for screening compounds and its salts that change the binding property between ligands and the G-protein-coupled receptor protein or its salts described in (1) in which the amount of a labeled ligand bound to cells containing the G-protein-coupled receptor protein described in (1) is measured after (i) contacting the labeled ligand to the cells and compared with that after (ii) contacting the labeled ligand and a test compound to the cells containing the G-protein-coupled receptor protein described in (1).
(34) The method for screening compounds and its salts that change the binding property between ligands and the G-protein-coupled receptor protein or its salts described in (1) in which the amount of a labeled ligand bound to cell membrane fraction containing the G-protein-coupled receptor protein described in (1) is measured after (i) contacting the labeled ligand to said cell membrane fraction and compared with that after (ii) contacting the labeled ligand and a test compound to said cell membrane fraction containing the G-protein-coupled receptor protein described in (1).
(35) The method for screening compounds and its salts that change the binding property between ligands and the G-protein-coupled receptor protein or its salts described in (1) in which the amount of a labeled ligand bound to the G-protein-coupled receptor protein is measured after (i) contacting the labeled ligand to the G-protein-coupled receptor protein expressed on the cell membranes of transformant induced by culturing said trasnformant described in (7) and compared with that after (ii) contacting the labeled ligand and a test compound to the G-protein-coupled receptor protein expressed on the cell membranes of said transformants induced by culturing said trasnformant described in (7).
(36) The method for screening compounds and its salts that change the binding property between ligands and the G-protein-coupled receptor protein or its salts described in (1) in which (i) the cell stimulatory activity via the G-protein-coupled receptor protein is measured after contacting a compound that activates the G-protein-coupled receptor protein or its salts described in (1) to cells containing the G-protein-coupled receptor protein described in (1) and compared with that (ii) after contacting said compound to said cells containing the G-protein-coupled receptor protein described in (1).
(37) The method for screening compounds and its salts that change the binding property between ligands and the G-protein-coupled receptor protein in which the cell stimulatory activity via the G-protein-coupled receptor protein is measured after contacting a compound that activates the G-protein-coupled receptor protein or its salts described in (1) to the G-protein-coupled receptor protein expressed on the cell membrane of cultured transformant described in (7) and compared with that after contacting said compound and a test compound to the G-protein-coupled receptor protein expressed on the cell membrane of said cultured transformants described in (7).
(38) The screening methods described in (36) or (37) in which compounds that activate the G-protein-coupled rereptor protein described in (1) are angiotensin, bombesin, canavinoid, cholecystokinin, glutamine, serotonin, melatonin, neuropeptide Y, opioids, purines, vasopressin, oxytocin, PACAP, secretin, glucagon, calcitnonin, adrenomedulin, somatostatin, GHRH, CRF, ACTH, GRP, PTH, vasoactive intestinal and related polypeptide (VIP), somatostatin, dopamine, motilin, amyrin, bradykinin, calcitonin gene-related peptide (CGRP), leukotriens, pancreastatin, prostaglandins, thromboxane, adenosine, adrenaline, α and β-chemokines (e.g. IL-8, GROα, GROβ, GROγ, NAP-2, ENA-78, PF4, IP10, GCP-2, MCP-1, HC14, MCP-3, I-309, MIP1α, MIP-1β, and RANTES), endothelin, enterogastrin, histamine, neurotensin, TRH, pancreatic polypeptide, and galanin.
(39) Compounds or its salts that change the binding property between ligands obtained using the methods for screening described in (31) - (38) and the G-protein-coupled receptor protein or its salts described in (1).
(40) Pharmaceuticals characterized by containing compounds or its salts that change the binding property between ligands obtained using the screening methods described in (31) - (38) and the G-protein-coupled receptor protein or its salts described in (1).
(41) The sreening kits described in (16) characterized by containing cells containing the G-protein-coupled receptor protein described in (1).
(42) The sreening kits described in (16) characterized by containing cell membrane fraction containing the G-protein-coupled receptor protein described in (1).
(43) The sreening kits described in (16) characterized by containing the G-protein-coupled receptor protein expressed on the cell membranes of cultured transformant described in (7).
(44) Compounds or its salts that change the binding property of ligands obtained using the screening kits described in (41) - (43) and the G-protein-coupled receptor protein or its salts described in (1).
(45) Pharmaceuticals characterized by containing compounds or its salts that change the binding property of ligands obtained using the screening kits described in (41) - (43) and the G-protein-coupled receptor protein or its salts described in (1).
(46) Methods for quantifying the G-protein-coupled receptor protein or its salts described in (1) or the partial peptides and salts thereof described in (2) characterized by contacting the antibodies described in (9) with the G-protein-coupled receptor protein or its salts described in (1) or the partial peptides or salts thereof described in (2).
(47) Methods for quantifying the G-protein-coupled receptor protein or its salts described in (1) and the partial peptides or its salts described in (2) in test solutions in which the ratio of the G-protein-coupled receptor protein or its salts described in (1) or the partial peptides or salts thereof described in (2) labeled with the antibody described in (9) is measured after competitive reaction among said antibody, the test solution, and the G-protein-coupled receptor protein or its salts described in (1) or the partial peptides or salts thereof described in (2); and
(48) Methods for quantifying the G-protein-coupled receptor protein or its salts described in (1) and the partial peptides or its salts described in (2) in test solutions in which a test solution, the antibody described in (9) immobilized on a carrier, and labeled said antibody described in (9) are simultaneously or sequenctially reacted, and the activity of the label on the immobilizing carrier is measured.

### BRIEF DESCRIPTION OF THE DRAWINGS

Figure 1 shows the base sequence of DNA enconding the rat cerebellum-derived novel G-protein-coupled receptor protein rCB7T084 of this invention obtained in Example 1, and the amino acid sequence deduced from the DNA sequence (continued to Fiure 2).

Figure 2 shows the base sequence of DNA enconding the rat cerebellum-derived novel G-protein-coupled receptor protein rCB7T084 of this invention obtained in Example 1, and the amino acid sequence deduced from the DNA sequence (continued from Fiure 1).

Figure 3 shows the hydrophobicity plot of the rat cerebellum-derived G-protein-coupled receptor protein rCB7T084 of this invention prepared based on the amino acid sequence shown in Figures 1 and 2.

### BEST MODE OF EMBODIMENT OF THE INVENTION

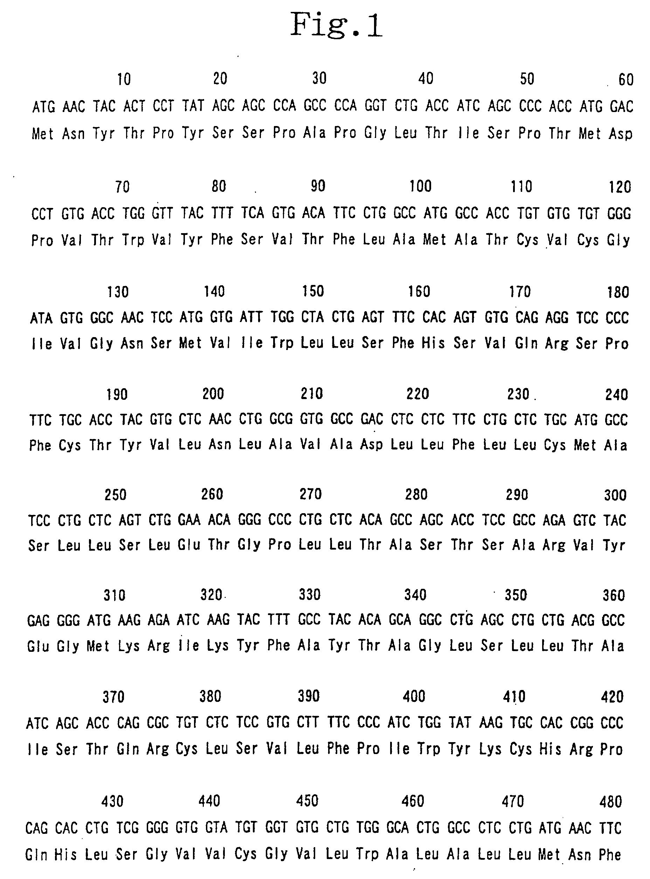

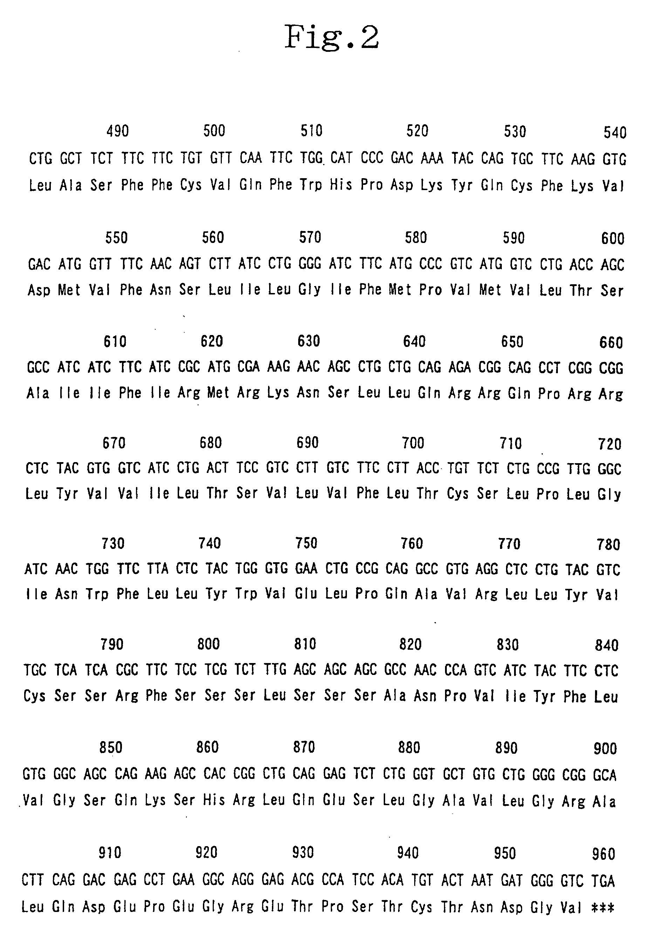

The G-protein-coupled receptor protein (receptor protein) of this invention is a receptor protein containing identical or substantially identical to the amino acid sequence presented by SEQ ID :1 (amino acid sequences shown in Figures 1 and 2).

The receptor protein of this invention may be derived from any type of cells of humans and mammals (e.g. guinea pigs, rats, mice, rabbits, pigs, sheep, cattle, monkeys, others), for example, splenocytes, neurons, glia cells, pancreatic â cells, bone marrow cells, mesangium cells, Langerhans cells, epidermal cells, epithelial cells, endothelial cells, fibroblasts, fibre cells, muscle cells, adipocytes, immune cells (e.g. macrophages, T cells, B cells, natural killer cells, mast cells, neutrophils, basophils, eosinophils, monocytes), megakaryocytes, synovial cells, chondrocytes, osteocytes, osteoblasts, osteoclasts, ,mammary cells, hepatocytes, and interstitial cells, precursor cells, stem cells and cancer cells of said cells, and cells in the blood cell system. The receptor protein may also derived from any tissue in which said cells are present, for exmaple, the brain, each region of the brain (e.g. olfactory bulbs, amyglada, basal ganglia, hippocampus, thalamus, hypothalamus, subthalamic nucleus, cerebral cortex, medulla oblongata, cerebelleum, occipital lobes, frontal lobe, lateral lobe, putamen, caudate nucleus, corpus callosum, substantia nigra), spinal cord, pituitary gland, stomach, pancreas, kidneys, liver, gonads, thyroid gland, gallbladder, bone marrow, adrenal glands, skin, muscle, lung, digestive tract (e.g. large intestine, small intestine), vascular vessels, heart, thymus, spleen, submandibular gland, peripheral blood, peripheral blood cells, prostate, orchis, testes, ovaries, placenta, uterus, bones, joints, and skeletal muscles (especially, the brain and each region of the brain). The receptor protein may also be synthetic.

Amino acid sequences substantially identical to the amino acid sequence presented by SEQ ID:1 are amino acid sequences having about 50% or more homology, preferably about 70% or more homology, more preferably about 80% or more homology, and further more preferably about 90% or more homology, and most preferably about 95% or more homogoly to the amino acid sequences presented by SEQ ID:1.

As proteins containing amino acid sequences substantially identical to the amino acid sequence of this invention presented by SEQ ID:1, for example, proteins that contain amino acid sequences that are substantially identical to the amino acid sequence presented by SEQ ID:1 and have substantially the same activity as that of the amino acid presented by SEQ ID:1 are preferred.

As to the substatially same activity, ligand-binding property and signal transduction activity are included. 'Substatially same' means that the quality of the activity is same. Therefore, although it is preferable that the activities such as ligand-binding and signal transduction activities (e.g. about 0.01- to 100-fold, preferably about 0.5- to 20-fold, more preferably about 0.5- to 2-fold)are equivalent, quantitative factors such as the level of activity and the molecular weight of the protein may differ.

The ligand-binding property and signal transduction acitivity can be measured according to publicly known methods, but these activities can be measured accordng to the method for deermining ligand and the method for screening described below.

Proteins containing the following amino acid sequences are used as the receptor protein of this invention: ① amino acid sequences presented by SEQ ID:1 in which one, two, or more amino acids (preferably 1 - 30 amino acids, more preferably 1 - 10 amino acids, most preferably several (1 - 5) amino acids) are deleted; ② amino acid sequences presented by SEQ ID:1 in which one, two, or more amino acids (preferably 1 - 30 amino acids, more preferably 1 - 10 amino acids, and most preferably several (1 - 5) amino acids) are added; ③ amino acid sequences presented by SEQ ID:1 in which one, two, or more amino acids (preferably 1 - 30 amino acids, more preferably 1 - 10 amino acids, and most preferably several (1 - 5) amino acids) are substituted by other amino acids; and ④ combinations of the amino acid sequences described in ① to ④ .

In this specification, the receptor protein is presented according to the conventinal presentation manner of peptides: the left end presents the N terminal (amino terminal) and the right end presents the C terminal (carboxyl terminal). In the receptor proteins of this invention including the receptor protein containing the amino acid sequence presented by SEQ ID:1, the C terminal is usually carboxyl group (-COOH) or carboxylate (-COO⁻), but the C terminal may be amide (-CONH₂) or ester (-COOR).

For R in the esters, C₁₋₆ alkyl groups such as methyl, ethyl, n-propyl, isopropyl, and n-butyl, C₃₋₈ cycloalkyl groups such as cyclopentyl and cyclohexyl, C₆₋₁₂ aryl groups such as phenyl and α-naphthyl, C₇₋₁₄ aralkyl groups including C₁₋₂ alkyl groups such as benzyl and phenethyl or α-naphthyl-C₁₋₂ alkyl groups such as α-naphthylmethyl are used, and pivaloyloxymethyl groups, which are commonly used for oral esters, is also used.

When the receptor protein of this invention has a carboxyl group (or carboxylate) at a site other than the C terminal, the receptor proteins having an amidized or esterified caroboxyl group are included in the receptor proteins of this invention. For the ester form in this case, for example, the C-terminal esters described above are used.

The receptor proteins of this invention also include proteins described above in which the amino group of the N-terminal methionine residue is protected by a protecting group (e.g. C₁₋₆ acyl group of C₂₋₆ alkanoyl group such as formyl group and acetyl), those in which the N-terminal is cleaved in vivo and the glutamyl group producd is substituted to pyroglutamate, those in which substituents in amino aicd side chains in the molecule (e.g. -OH, -SH, amino group, imidazole group, indole group, guanidino group) are protected by appropriate protecting groups (e.g. C₁₋₆ acyl group of C₂₋₆ alkanoyl group such as formyl group and acetyl), or complex proteins to which sugar chains are bound, that is glycoproteins.

As a specific example of the receptor protein of this invention, the rat-derived (preferably rat cerebellum-derived) receptor protein containing the amino acid sequence presented by SEQ ID:1 is used.

For the partial peptides of the receptor protein of this invention (partial peptides), any partial peptide of the receptor ptotein of this invention described above may be used. For example, among the receptor protein molecules of this invention, the region exposed to outside of the cell membrane having the receptor-binding acitvity is used.

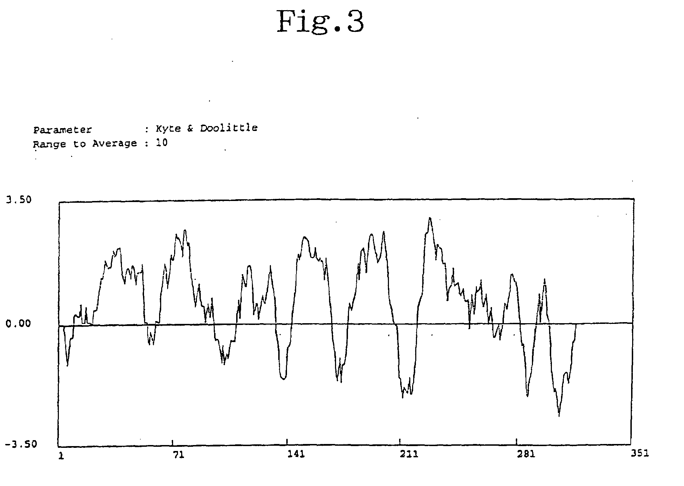

Concretely, the partial peptides of the receptor protein presented by Seqeunce Number:1 are peptides containing the regions that were shown to be the extracellular domains (hydrophilic regions) by the hydrophobicity plot analysis shown in Figure 3. Peptides containing a part of hydrophobic region may also be used. Peptides containing individual domains can be used, but peptides containing multiple domains may also be used.

The number of amino acids in the partial peptides of this invention is at least 20 or more, preferably 50 or more, and more preferably 100 or more of the constitutive amino acid sequence of the receptor protein of this invention described above.

The substatially identical amino acid sequences are amino acid sequences that have about 50% or more, preferably about 70% or more, more preferably about 80% or more, furthermore preferably about 90% or more, and most preferably 95% or more homology to the amino acid sequences described above.

'Substaitially same activity' means the same definition descriebd above. 'Substiantially same acitivity' can be measured as described above.

In the partial peptides of this invention, one, two, or more amino acids (preferably 1 - 10 amino acids, more preferably several (1 - 5) amino acids) may be deleted, one, two, or more amino acids (preferably 1 - 20 amino acids, preferably 1 - 10 amino acids, and more preferably several (1 - 5) amino acids) may be added, and one, two, or more amino acids (preferably 1 - 10 amino acids, more preferably several amino acids, and most preferably 1 - 5 amino acids) may be substituted by other amino acids.

In the partial peptides of this invention, the C-terminal is usually carboxyl group (-COOH) or carboxylate (-COO⁻), but the C-terminla may be amide (-CONH₂) or ester (-COOR) as in the proteins of this invention described above.

As the receptor proteins of this invention described above, the partial peptides of this invension include peptides in which the amino group of the N-terminal methione residue is protected by a protecting group, those in which the N-terminal residue is cleaved in vivo and the Gln produced is changed to pyroglutamate, those in which substituents in amino acid side chains in the molecule are protected by appropriate protecting groups, or those in which sugar chains are bound, that is golycopeptides.

In the partial peptides of this invention, the C-terminal is usually carboxyl group (-COOH) or carboxylate (-COO'), but the C-terminal may be amide (-CONH₂) or ester (-COOR), as in the proteins of this invention described above.

The salts of the receptor proteins or partial peptides of this invention include physiologically acceptable salts formed with acids or bases, especially physiologically acceptable salts formed with acids are preferred. For examples, the salts formed with inorganic acids (e.g. hydrochloric acid, phosphoric acid, hydrobromic acid, sulfuric acid) and the salts formed with organic acids (e.g. acetic acid, formic acid, propionic acid, fumaric acid, maleic acid, succinic acid, tartaric acid, citric acid, malic acid, oxalic acid, benzoic acid, methanesufonic acid, benzenesulfonic acid) are used.

The receptor proteins and its salts of this invention can be manufactured by the publicly known method for purifying receptor protein from the human and mammalian cells or tissues described above. They may also be manufactured by culturing transformants containing the DNA encoding the receptor proteins of this invention described below, and by the protein synthesis method described below or the modified method.

When the receptor protein is produced from human or mammlian tissues or cells, human or mammalian tissues or cells are homogenized, followed by extracting with acid or the like, and the extract is subjected to a combination of chromatography such as reversed-phase chromatogtraphy and ion-exchange chromatography to isolate and purify the receptor protein.

For synthesis of the receptor proteins, partial peptides, or its salts and amide forms of this invention, commercially available resins for protein synthesis can be used. Such resins include chloromethyl resin, hydroxymethyl resion, benzhydrylamine resin, aminomethyl resin, 4-benzyloxybenzyl alcohol resin, 4-methylbenzhydrylamine resin, PAM resin, 4-hydroxymethylmehtyl phenyl acetoamidomethyl resin, polyacrylamide resin, 4-(2',4'-dimethoxyphenyl-hydroxymethyl)phenoxy resin, and 4-(2',4'-dimethoxyphenyl-Fmoc aminoethyl) phenoxy resin. Using these resins, amino acids in which the α-amino groups and the side-chain functional groups appropriately protected are condensed in the order of the sequecne of the objective protein on the resin according to the various publicly known condensation methods. At the end of the reaction, the protein is excised from the resin and the protecting groups are simultaenosuly removed. Then, intramolecular disufide bond-forming reaction is performed in a highly diluted solution to obtain the objective protein or its amide form.

For condensation of the protected amino acids described above, various activation reagent for protein synthesis can be used, but carbodiimides are particularly good. For carbodiimides, DCC, N,N'-diisopropylcarbodiimide, and N-ethyl-N'-(3-dimethylaminoproryl)carbodiimide are used. For activation by these reagents, the protected amino acids are added with a racemization inhibitor (e.g. HOBt, HOOBt) directly to the resin, or the protected amino acids are previously activated as corresponding acid anhydrides, HOBt esters, or HOOBt esters, then added to the resin.

The solvent used for activation of the protected amino acids and condensation with the resin can be selected from solvents known to be useful in protein condensation reaction. For example, acid amides such as N,N-dimethylformamide, N,N-dimethylacetoamide, and N-methylpyrrollidone, hydrocarbon halogenides such as methylene hydrochloride and chloroform, alcohols such as trifluoroethanol, sulfoxide such as dimethylsufoxide, ethers such as pyridine, dioxane, and tetrahydrofuran, nitriles such as acetonitrile and propionitrile, esters such as methyl acetate and ethyl acetate, or appropriate mixtures of these solvents. The appropriate reaction temperature is selected from the range known to be used in protein bonding reaction, and usually selected from the range from about -20°C to 50°C. The activated amino acid derivatives are usually used in 1.5- to 4-fold excess. The condensation is tested using ninhydrin reaction, and when the condensation is not sufficient, sufficient condensation can be obtained by repeating the condensation reaction without elimination of the protecting groups. When the condensation is insufficient even after repeating the reaction, non-reacted amino acids can be acetylated using acetic anhydride or acetylimidazole.

For the protecting groups for amino acids of raw material, for examples, Z, Boc, tertiary pentyloxycarbonyl, isobornyloxycarbonyl, 4-methoxybenzyloxycarbonyl, Cl-Z, Br-Z, adamantyl oxycarbonyl, trifluoroacetyl, phthaloyl, formyl, 2-nitrophenylsulphenyl, diphenylphosphino thioyl, and Fmoc are used.

Carboxyl group can be protected by, for example, alkyl esterification (linear, branched, and cyclic alkyl esterification such as methyl, ethyl, propyl, butyl, tertiary butyl, cyclopentyl, cyclohexyl, cycloheptyl, cyclooctyl, and 2-adamantyl), aralkyl esterification (e.g. benzyl ester, 4-nitrobenzyl ester, 4-methoxybenzyl ester, 4-chlorobenzyl ester, benzhydryl ester), phenacyl esterification, benzyloxylcarbonyl hydrazidation, tertiary butoxycarbonyl hydrazidation, and trityl hydrazidation.

The hydroxyl group of serine can be protected, for example, by esterification or etherification. Groups appropriate for this esterification include, for example, short alkanoyl groups such as acetyl group, aroyl groups such as benzoyl group, and groups derived from carbonic acid such as benzyloxycarbonyl group and ethoxycarbonyl group. Groups appropriate for etherification include benzyl group, tetrahydropyranyl group, and t-butyl group.

As a protecting group of the phenolic hydroxyl group of tyrosine, for example, BzL, Cl₂-Bzl, 2-nitrobenzyl, Br-Z, and tertiary butyl are used.

As a protecting group of the imidazole of histidine, for example, Tos, 4-methoxy-2,3,6-trimethylbenzen sulfonyl, DNP, benzyloxymethyl, Bum, Boc, Trt, and Fmoc are used.

For activated carboxyl groups in the raw material, include corresponding acid anhydride, azide, active ester [ester formed with alcohol (e.g. pentachlorophenol, 2,4,5-trichlorophenol, 2,4-dinitrophenol, cyanomethyl alcohol, paranitrophenol, HONB, N-hydroxysuccimide, N-hydroxyphthalimide, HOBt)] are used. Activated amino groups used in the raw material include corresponding phosphoric amide.

For the method for eliminating the protecting groups, catalytic reduction in hydrogen gas flow in the presence of a catalyst such as Pd-black and Pd-carbon, acid treatment with hydrogen fluoride anhydride, methanesulfonic acid, trifluoromethanesulfonic acid, and trofluoroacetic acid, and mixture of these acids, basic treatment with diisopropylethylamine, triethylamine, piperidine, and piperazine, and reduction by sodium in liquid anmmonia are used. The elimination reaction by the acid treatment described above is generally performed at a temperature ranging from about -20°C to 40°C. In acid treatment, addition of a cation scavenger such as anisole, phenol, thioanisole, metacresol, paracresol, dimethylsulfide, 1,4-butanedithiol, and 1,2-ethanedithiol is effective. 2,4-dinitrophenyl group used as the protecting group of the imidazole of histidine is removed by treatment with thiophenol. Formyl group used as the protecting group of the indole of tryptophan is removed by the acid treatment in the presence of 1,2-ethanedithiol and 1,4-butanedithiol, as well as alkaline treatment with diluted sodium hydroxide solution and diluted ammonia.

Protection of functional groups that should not be involved in the reaction of the raw materials, protecting groups, elimination of the protecting groups, and activation of functional groups involved in the reaction may be appropriately selected from publicly known groups and means.

In another method for obtaining amide form proteins, for example, first, the α-carboxyl group of the carboxy termial amino acid is protected by amidation, and the peptide (protein) chain is extended for a desried length from the amino group side. Then, a protein in which only the protecting group of the N-terminal α-amino group was removed from said peptide and a protein in which only the protecting group of the C-terminal carboxyl group is removed are produced. These two proteins are condensed in the mixed solvent described above. The details of the condensation reaction are the same as described above. After the protected protein obtained by condensation is purified, all protecting groups are removed by the method described above, and the desired crude protein is obtained. The desired protein in amide form can be obtained by purifying this crude protein using various known means and by lyophilizing the major fraction..

To obtain esterified protein, for example, the α-carboxyl group of the carboxy termial amino acid is condensed with a desired alcohol to prpare amino acid ester, and the desired esterified protein can be obtained by the same procedure as in the preparation of the amide form of protein.

The partial peptides or its salts of this invention can be manufactured by publicly known method for peptide synthesis or by cleaving the protein of this invesntion with appropriate peptidase. For the method for peptide synthesis, for example, either solid phase synthesis or liquid phase synthesis may be used. The partial peptides or amino acids that may compose the protein of this invention are condensed with the residual portion. When the product has protecting groups, the desired peptide can be obtained by eliminating the protecting groups. The publicly known condensation and elimination methods include the methods described in 1) - 5) below.
1) M. Bodanszky and M.A. Ondetti: Peptide Synthesis. Interscience Publishers, New York (1966)
2) Schroeder and Luebke: The Peptide. Academic Press, New York (1965)
3) N. Izumiya, et al.: Basics and experiments of peptide synthesis, Maruzen Co. (1975)
4) H. Yajima and S. Sakakibara: Biochemical Experiment 1, Chemistry of Proteins IV, 205 (1977)
5) H. Yajima ed.: A sequel to Development of Pharmaceuticals Vol. 14, Peptide Synthesis, Hirokawa Shoten

After the reaction, the partial peptides of this invention are purified by a combination of conventional purification methods such as solvent extraction, distillation, column chromatography, liquid chromatography, and recrystallization. When the partial peptide obtained by the above methods is free form, it can be converted to an appropriate salt form by known methods. On the oher hand, when a salt form is obtained, it can be converted to the free form by known methods.

For the polynucleotide encoding the receptor ptotein of this invention, any polynucleotide containing the base sequence (DNA or RNA, preferably DNA) encoding the receptor protein of this invention described above can be used. Said polynucleotide may be DNA and RNA including mRNA encoding the receptor protein of this invetion, in which may be double-stranded or single-stranded. When the polynucleotide is double-stranded, it may be double-stranded DNA, double-stranded RNA, or DNA:RNA hybrid. When the polunucleotide is single-stranded, it may be sense strand (i.e. coding strand) or antisense strand (i.e. non-conding strand).

Using the polynucleotide encoding the receptor protein of this invention, mRNA of the receptor protein of this invention can be quantified by, for example, the known method published in separate volume of Jikken Igaku 15 (7) 'New PCR and its application' (1997) or the modified method.

The DNA encoding the receptor protein of this invention may be any DNA of genomic DNA, genomic DNA library, cDNA derived from the cells and tissues described above, and synthetic DNA. For the vector used for library, bacteriophage, plasmid, cosmid, and phagemid may be used. The DNA may be directly amplified by reverse transcriptase polymerase chain reaction (RT-RCR) using total RNA or mRNA fraction prepared from the cells and tissues described above.

Concretely, for the DNA encoding the receptor protein of this invention, for example, DNA containing the base sequence presented by SEQ ID:2, and any DNA encoding the receptor protein of this invention that contains a base sequence that hybridize to the base sequence presented by SEQ ID:2 under a high stringent condition and have substantially the same activity as that of the receptor protein of this invention (e.g. ligand-binding activity and signal transduction activity).

For the DNA that hybridizes to the base sequence presented by SEQ ID:2, for example, DNAs containing a base sequence that have about 70% or more homology, preferably about 80% or more homology, more preferably about 90% or more homolgy, and most preferably about 95% or more homology to the base sequence presented by SEQ ID:2 are used.

Hybridization can be performed using publicly known method or the modified method such as the method described in Molecular Cloning, 2nd (J. Sambrook et al., Cold Spring Harbor Lab. Press, 1989). When a commercial library is used, hybridization may be performed according to the attached instruction. Preferably, hybridization may be performed under a high stringent condition.

In said high stringent condition, for example, the sodium concentration is about 19 - 40 mM, preferably about 19 - 20 mM, and the temperature is about 50 - 70°C, preferably about 60 - 65°C. In the most preferred condition, the sodium concentration is about 19 mM and the temperature is about 65°C.

More concretely, for the DNA encoding the receptor protein having an amino acid sequence presented by SEQ ID:1, the DNAs encoding the base sequence presented by SEQ ID:2 are used.

The polynucleotides containing a part of the base sequence of the DNA encoding the receptor of this invention or a part of the base sequence complementary to the DNA include not only DNAs encoding the partial peptides of this invention described below but also the RNAs.

According to the present invention, antisense polynucleotides (nucleic acids) that inhibit replication or expression of the G-protein-coupled receptor protein gene can be designed and synthesized based on the cloned or the detemined base sequence information of the DNA encoding the G-protein-coupled receptor protein. Such polynucleotides (nucleic acids) can hybridize to the RNA of the G-protein-coupled receptor protein, and inhibit the synthesis or function of the RNA, or regulate/control the expression of the G-protein-coupled receptor protein gene via interaction with RNAs related to the G-protein-coupled receptor protein. Polynucletides complenetary to specified sequences of RNAs related to the G-protein-coupled receptor protein and polynucleotides that specifically hybridize to RNAs related to the G-protein-coupled receptor protein are useful for regulation/control of the expression of the G-protein-coupled receptor protein gene in vivo and in vitro, and are also useful for treatment or diagnosis of diseases. The term 'correspond' means 'homologous' or 'complementary' to a specific sequence of nucleotides including the gene, base sequences, and nucleic acids. The term 'correspond' among nucleotide, base sequence, or nucleic acid, and peptides (proteins) generally means that the amino acids of the peptide (protein) is to be induced from the nucleotide (nucleic acid) sequence or its compelmetary sequence. The 5'-end heairpin loop, 5'-end 6 base pairs-repeat, 5'-end non-translational region, polypeptide transplation initiation codon, protein-encoding region, ORF translation initiation codon, 3'-end non-transplational region, 3'-end palindrome region, and 3'-end hairpin loop of the G-protein-coupled receptor protein gene may be selected as a preferable region, but any region within the G-protein-coupled receptor protein gene may be selected.

The relationship between the objective nucleic acid and the polynuclotide compelementary to at least a part of the subject region is defined as 'antisence'. Antisense polynucletide includes polydeoxynucleotide containing 2-deoxy-D-ribose, polydeoxynucleotide containing D-ribose, other types of polyunucleotide that are N-glycoside of purine or pyrimidine base, other polymers having a non-nucleotide back bone (e.g. commercial protein nucleic acids, and nucleic acid polymers specific for synthetic sequences) or other polymers containing specific bonds (said polymers contain a nucleotide sequence having a structure that accepts base pairing or attachment found in DNA or RNA). Said antisense polynucleotides may be double-stranded DNA, single-stranded DNA, double-stranded RNA, single-stranded RNA, and DNA:RNA hybrid. Said antisense polynucleotides may also be non-modified polynucletide (or non-modified oligonucleotide), may be added with publicly known modification such as labels known in the art, capping, methylation, and substitution of one or more natural nucleotide by analogues, may be intramolecular-modified nucleotides such as non-charged bonds (e.g. methylphosphonate, phosphotriester, phosphoramidate, carbamate), may contain charged bonds or sulfur-containing bonds (e.g. phosphorothioate, phosphorodithioate), may have side-chain groups such as proteins (e.g. nuclease, nuclease inhibitor, toxin, antibody, signal peptide, poly-L-lysine) and sugars (e.g. monosacchrides), may contain intercalating compound (e.g. acridine, psolarene), may contain chelate compound (e.g. metals, radioactive metals, boron, oxidative metals), may contain alkylating agent, and may contain modified bonds (e.g. α-anomeric nucleic adids), wherein 'nucleoside,' 'nucleotide,' and 'nucleic acid' may include not only purine and pyrimidine bases but also other modified heterocyclic bases. Such modified bases may be methylated purine and pyrimidine, acylated purine and pyrimidine, and other heterocycles. The modified nucleotide and modidifed nucleic acid may be modified at the glycosyl region, for example, one or more hydroxyl groups may be substituted by halogens or aliphatic groups or converted to functional groups such as ether and amine.

The antisense polynucleotides (nucleic acids) of this invention are RNA, DNA, or modified nucleic acids (RNA, DNA). Modified nucleic acids include sulfur derivatives and thiophosphate derivatives of the nucleic acid and nucleic acids resistant to degradation of the polynucleoside amide or oligonucleoside amide, but are not liminted to these molecules. The antisense nucleic acids of this invention are preferably designed following the policies below: The antisense nucleic acids are desinged to have more intracellular stability, to have increased cell permeability, to have increased affinity for the objective sense strand, and to have decreased txocity, if it has toxicity.

There are many such modifications known in the art. For example, modifications are disclosed in Pharm Tech Japan, Vol. 8, pp. 247, 1992 and Vol. 8, pp. 395, 1992 by J. Kawakami et al., and in S.T. Crooke et al. ed., Antisense Research and Applications, CRC Press, 1993.

The antisense nucleic acids of this invention may be changed or may contain modified sugars, bases, and bonds, may be provided in specific forms such as liposomes and microspheres, and may be applied to gene therapy or applied in additive forms. Such additive form includes hydrophobic form such as polycationic forms e.g. polylysine that neutralizes the charge of the phosphate backbone, lipids that increase the interaction with cell membranes or nucleic acid uptake (e.g. phospholipids, choresterol). Lipids preferable to add include choresterol and its derivatives (e.g. chresterylformate, cholic acid). These forms can be attached to the 3'- or 5'-end of the nucleic acid via base, sugar, and intramolecular nucloside bonding. Other groups are for capping specifically positioned at the 3'- or 5'-end of the nucleic acid, including molecules that prevent degradation by nucleases such as exonuclease and RNase. The groups for capping include protecting groups of hydroxyl group in the art such as glycols (e.g. polyehtyleneglycol, tetraethyleneglycol), but are not limited to these protecting groups.

The inhibition activity of the antisense nucleic acids can be examined using the transformants of this invention, the in vivo and in vitro gene expression systems of this invention, or the in vivo and in vitro translation systems of the G-protein-coupled receptor protein. Said nucleic acids can be directly applied to cells using various known methods.

The DNAs encoding the partial peptides of this invention may be any molecule that contains a base sequence enconding the partial peptide of this invention, and may be genomic DNA, genomic DNA library, cDNA and cDNA library derived from the cells and tissues described above, and synthetic DNA. Vectors used for library may be bacteriophage, plasmid, cosmid, and phagemid. The DNA may be directly amplified using mRNA fraction prepared from the cells and tissues described above by reverse transcriptase polymerase chain reaction (RT-PCR).

Concretely, as to the DNA encoding the partial peptides of this invention are, for example: (1) DNA containing a portion of the base sequence of the DNA presented by SEQ ID:2, and (2) DNA that contains a base sequence that hybridizes to the base sequence presented by SEQ ID:2 under a high stringent condition and a portion of the DNA sequence encoding the receptor protein peptide having substantially same activity (e.g. ligand-binding property, singal transduction activity) to the activity of the receptor protein peptide of this invention are used.

For the DNAs that hybridized to the base sequence presented by SEQ ID:2, DNAs containing about 70% or more, preferably about 80% or more, more preferably about 90% or more, and most preferably about 95% or more homology to the base sequence presented by SEQ ID:2 are used.

For the method for cloning the DNA encoding the compelete receptor protein or its partial peptides (receptor proteins of this invention) of this invention, the DNA is amplified by PCR using synthetic DNA primers containing a part of the base sequence of the receptor protein of this inveniton, or the DNA inserted in an appropriate vector can be selected by hydridization with the labeled DNA fragment encoding a part or entire region of the receptor protein of this invention or synthetic DNA. Hybridization can be performed, for exmaple, by the method described in Molecular Cloning, 2nd (J. Sambrook et al., Cold Spring Harbor Lab. Press, 1989). Hybridization can also be performed when commercial libraly are used according to the method described in the attached instruction.

Conversion of the DNA sequences can be performed by publicly known methods such as Gupped duplex method and Kunkel method or its modified methods using a known kit such as Mutan^{TM}-G (Takara Shuzo Co.) or Mutan™-K (Takara Shuzo Co.).

The cloned DNAs encoding the receptor protein can be used without treatment or used after digestion with restriction enzymes or addition of linkers when necessary. Said DNA may contain the translational initiation codon ATG at the 5'-end and transolational stop codon TAA, TGA, or TAG at the 3'-end. These translational initiation codon and stop codon can be added using an appropriate synthetic DNA adaptor.

Expression vectors for the receptor protein of this invention can be manufactured, for example, as follows: (i) The objective DNA fragment is excised from the DNA encoding the repceptor protein of this inveniton, and (ii) the DNA fragment is ligated to downstream of the promoter in an appropriate vector.

For the vector, *Escherichia coli*-derived plasmid (e.g. pBR322, pBR325, pUC12, pUC13), *Bacillus subtilis-* derived plasmid (e.g. pUB110, pTP5, pC194), *yeast-* drived plasmid (e.g. pSH19, pSH15), bacteriophages such as λ phage, animal viruses such as retrovirus, vaccinia virus, and baculovirus, and pAl-11, pXT1, pRC/CMV, pRC/RSV, and pcDNAI/Neo are used.

Any promoter that is appropriate and coppresponds to the host used for the gene expression may be used as the promoter used in this invention. For example, when animl cells are used as the host, SRα promoter, SV40 promoetr, LTR promoter, CMV promoter, and HSV-TK promoter are used. Among them, CMV promoter and SRα promoter are preferred. When the host is bacteria of *Escherichia genus,* trp promoter, lac promoter, recA promoter, λP_{L} promoter, and lpp promoter are preferred. When the host is bacteria of *Bacillus genus,* SPO1 promoter, SPO2 promoter, penP promoter are preferred. When the host is yeast, PHO5 promoter, PGK promoter, GAP promoter, and ADH promoter are preferred. When the host is insect cells, polyhedrin promoter and P10 promoter are preferred.

In addition to the vectors described above, expression vectors contaninig enhancer, splicing signal, polyA signal, selection marker, and SV40 replication origin (SV40 ori) may be used when desired. For the selection marker, for example, dihydrofolate reductase (dhfr) gene [methotrexate (MTX)-resistant], ampicillin resistance gene (Amp^{r}), and neomycin resistance gene (Neo^{r}, G418-resistant) are used. Especially, when dhfr gene is used as the selection marker using CHO cells (dhfr⁻), the objective gene can be selected using thymidine-free medium.

When necessary, a signal sequence appropriate for the host is added to the N-terminal of the receptor protein of this invention. When the host is bacteria of *Escherichia genus,* PhoA signal sequence and OmpA signal sequence are used. When the host is bacteria of *Bacillus genus,* α-amylase signal sequence and subtilisin signal sequence are used. When the host is yaest, MFα signal sequence and SUC2 signal sequence are used. When the host is animal cells, insulin signal sequence, α-inetrferon signal sequence, and the signal sequence of antibody molecule can be used.

Using the vectors containing the DNA encoding the receptor protein of this invention constructed as described above, transformants can be manufactured.

For the host, for exmaple, *Escherichia genus, Bacillus genus,* yeast, insect cells, insects, and animal cells are used.

Specific examples of the host of *Escherichia genus* are *Escherichia coli* K12 DH1 [Proceedings of the National Academy of Sciences of the USA (Proc. Natl. Acad. Sci. USA) Vol. 60, 160 (1968)], JM103 [Nucleic Acids Research Vol. 9, 309 (1981)], JA221 [Journal of Molecular Biology Vol. 120, 517 (1978)], HB101 [Journal of Molecular Biology Vol. 41, 459 (1969)], and C600 [Genetics Vol. 39, 440 (1954)] are used.

For the host of Bacillus genus, for example, *Bacillus subtilis* MI114 [Gene Vol. 24, 255 (1983)] and 207-21 [Journal of Biochemistry Vol. 95, 87 (1984)] are used.

For the host of yeast, for example, *Saccharomyces* *cerevisiae* AH22, AH22R⁻ , NA87-11A, DKD-5D, 20B-12, *Schizosaccharomyces* pombe NCYC1913, NCYC2036, and *Pichia pastoris* are used.

For the host of insect cells, for example, when the virus is AcNPV, Spodoptera frugiperda cells (Sf cells), MG1 cells derived from the middle gut of Trichoplusia ni, High Five™ cells derived from Trichoplusia ni eggs, Mamestra brassicae-derived cells, or Estigmena acrea-derived cells are used. When the virus is BmNPV, silkworm-derived cells Bombyx mori N (BmN cells) are used. For said Sf cells, for example, SF9 cells (ATCC CRL1711), Sf21 cells (Vaughn, J.L. et al., In Vivo 13, 213-217 (1977)) are used.

For the host of insect, for example, silkworm larvae are used [Maeda et al., Nature, Vol. 315, 592 (1985)].

For the host of animal cells, for example, monkey COS-7 cells, Vero, Chinese hamster CHO cells (CHO), dhfr gene-deficient Chinese hamster cells CHO (CHO (dhfr⁻) cells), mouse L cells, mouse AtT-20, mouse myeloma cells, rat GH3, and human FL cells are used.

For transformation of bacteria of *Escherichia genus,* for example, the methods published in Proc. Natl. Acad. Sci. USA Vol. 69, 2110 (1972) and Gene Vol. 17, 107 (1982) are used. Bacteria of *Bacillus genus* can be transformed according to, for example, the method published in Moelecular & General Genetics Vol. 168, 111 (1979).

Yeast can be transformed according to, for example, the methods published in Methods in Enzymology Vol. 194, 182-187 (1991) and Proc. Natl. Acad. Sci. USA Vol. 75, 1929 (1978).

Insect cells and insects can be transformed according to, for example, the method published in Bio/Technology, 6, 47-55 (1988).

Animal cells can be transformed by, for example, the methods described in Cell Engineering (Saibo Kogaku) Separate Vol. 8, New Cell Engineering Experimental Protocol, 263-267 (1995) (Shujun-sha) and Virology Vol. 52, 456 (1973).

As described above, the transformants transformed by the expression vector containing the DNA encoding the G-protein-coupled receptor protein are obtained.

For the medium for culturing the trasnformants using the *Escherichia* and *Bacillus* hosts, liquid medium is suitable, in which carbon source, nitrogen source, inorganic compounds, and other substances necessary for the growth of the transformants are contained. The carbon source includes glucose, dextrin, soluble starch, and sucrose. The nitrogen source includes inorganic and organic compounds such as ammonium salts, nitrates, corn steep liquor, peptone, casein, meat extract, soybean cake, and potato extract. The inorganic compounds include potassium chloride, sodium dihydrogen phosphate, and magnesium chloride. Yeast, vitamins, and growth-stimulating factors may be added. The pH about 5 - 8 is desirable for the culture.

For the culture medium for bacteria of *Escherichia genus,* for example, M9 medium containing glucose and casamino acids (Miller, Journal of Experiments in Molecular Genetics, 431-433, Cold Spring Harbor Laboratory, New York, 1972) is preferred. When more efficiency is required for the function of the promoter, reagent such as 3β-indoryacrylic acid may be added. When the host is bacteria of *Escherichia genus,* the bacteria are generally cultured at about 15 - 43°C for about 6 - 24 hours, and aeration or agitation may be added to the culture, when necessary.

When the host is bacteria of *Bacillus* genus, the bacteria are generally cultured at about 30 - 40°C for about 3 - 24 hours, and aeration or agitation may be added to the culture, when necessary.

For the medium for culturing the transformant of yeast host, for example, Burkholder minimum medium [Bostian, K.L. et al., Proc. Natl. Acad. Sci. USA Vol. 77, 4505 (1980)] and SD medium containing 0.5% casamino acid [Bitter, G.A. et al., Proc. Natl. Acad. Sci. USA Vol. 81, 5330 (1984)] are used. The pH of the medium is preferably adjusted to about 5 - 8. The culture are generally performed at about 20 - 35°C for about 24 - 72 hours, and aeration or agitation may be added to the culture, when necessary.

When the insect cells and insect derived transformants are cultured, Grace's insect medium (Grace, T.C.C., Nature, 195, 788 (1962)) containing appropriate supplements such as inactivated 10% bovine serum is used as a medium. The pH of the medium is preferably adjusted to about 6.2 - 6.4. Usually, the culture is performed at 27°C for about 3 - 5 days, and aeration or agitation may be added to the culture, when necessary.

When the transformants in animal cell host are cultured, for example, MEM containing about 5 - 20% fetal calf serum [(Sience Vol. 122, 501 (1952)), DMEM [Virology Vol. 8, 396 (1959)], RPMI 1640 medium [The Journal of the American Medical Association vol. 199, 519 (1967)], and 199 medium [Proceeding of the Society for the Biological Medicine Vol. 73, 1 (1950)] are used as a medium. The pH is preferably adjusted to about 6 - 8. Usually, the culture is performed at about 30 - 40°C for about 15 - 60 hours, and aeration or agitation may be added to the culture, when necessary.

Then, the G-protein-coupled receptor protein of this invention is produced on the cell membrane of the transformants.

The receptor protein of this invention can be purified from the culture described above by, for example, the methods described below.

When the receptor protein of this invention is extracted from the cultured bacteria or cells, the bacteria or cells are collected after culture by a publicly known method, and suspended in appropriate buffer. Then, the bacteria or cells are disrupted using ultrasonication, lysozymes, and/or by freezing-thawing, and the crude extract of receptor protein is obtained by centrifugation or filtration. The buffer may contain protein-denaturants such as urea and gauanidine hydrochloride and a surfactant such as Triton X-100™. When the receptor protein is secreted into the culture supernatant, the supernatnat is separated from the bacteria or cells after culture and collected using a publicly known method.

For purification of the receptor protein from the culture supernatnat or the extract obtained as described above, publicly known methods for separation and purification are appropriately combined. These publicly known methods for separation and purification include methods using solubility such as salting out and solvent precipitation, methods mainly using differences in molecular weight such as dialysis, ultrafiltration, gel filtration, and SDS-polyacrylamide gel electophoresis, methods using differences in electric charge such as ion-exchange chromatography, methods using specific affinity such as affinity chromatography, methods using differences in hydrophobicity such as reverse-phase high performance liquid chromatography, and methods using differences in isoelectric point such as isoelectric focusing.

When the receptor protein obtained is in the free form, the free form can be converted to salts by publicly known methods or its modified methods. On the other hand, when the receptor protein is obtained in a salt form, the salt form can be converted to the free form or other salts by publicly known methods or its modified methods.

The receptor protein produced by transformants can be optionally modified or partially removed a polypeptide from the receptor protein by treating the receptor protein with an appropriate protein-modifying enzyme before or after purification. For the protein-modifying enzyme, for example, trypsin, chymotrypsin, arginylendopeptidase, protein kinase, and glycosidase are used.

The activity of the receptor protein or its salts of this invention produced as described above can be measured by binding assay using labeled ligands and by enzyme immunoassay using specific antibody.

Antibodies against the receptor protein, its partial peptides, and its salts of this invention may be polycloncal antibody and monoclonal antibody that recognize the receptor protein of this invention, its partial peptides, and its salts.

Antibodies against the receptor protein, its partial peptides, and its salts of this invention (the receptor protein and its other forms of this invention ) can be manufactured according to publicly known methods for manufacturing antibody and antiserum using the receptor proteins and its other forms of this invention as antigens.

### [Preparation of monoclonal antibody]

### (a) Preparation of monoclonal antibody-producing cells

The receptor protein and its other forms of this invention is administered with or without carrier and diluent to sites by which antibody production is induced in mammals. To increase the antibody productivity, Freund complete or incomplete adjuvant may be administered. Usually, the protein will be administered every 2 - 6 weeks, 2 - 10 times in total. Mammals used include monkeys, rabbits, dogs, guinea pigs, mice, rats, sheep, and goats, and mice and rats are preferred.

In preparation of monoclonal antibody-producing cells, animals in which antibody titer is observed are selsected from warm-blooded animals immunized with antigen such as mice, and the spleen or lymph nodes are excised 2 - 5 days after the final immunization. Monoclonal antibody-producing hybridomas can be prepared by fusing the antibody-producing cells contained in the excised organ with myeloma cells. Antibody titer in an antiserum can be measured by, for example, reacting the labeled receptor protein or its other forms of this invention with the antiserum, then measuring the activity of the label bound to the antioby. The cell fusion can be performed according to known methods such as the method for Kohler and Milstein [Nature Vol. 256, 495 (1975)]. For the fusion-promoting agent, for example, polyethyleneglycol (PEG) and Sendai virus are used, and PEG is preferred.

For myerloma cells, for example, NS-1, P3U1, and SP2/0 are used, and P3U1 is preferred. The preferable ratio of the number of antibody-prudcing cells (spleen cells) to that of myeloma cells is 1:1 - 20:1. PEG (preferably PEG1000 - PEG6000) is added at about 10 - 80% concentration and the cells are incubated at about 20 - 40°C, preferably about 30 - 37°C, for about 1 - 10 minutes, then, an efficient cell fusion can be prepared.

Various methods for screening moclonal antibody-producing hybridomas ca be used. For example, hybridoma culture supernatant is added to antigen of the receptor protein or its other forms adsorbed to solid phase (e.g. microplate) directly or with carrier, then, anti-immunoglobulin antibody labeled with radioactive substance or enzyme (when the cells for fusion are mouse cells, anti-mouse immunogloblin antibody is used) or protein A is added, and the monoclonal antibody bound to the solid phase is detected. In other method, hybridoma culture supernatnat is added to anti-immunoglobulin antibody or protein A adsorbed to solid phase, then; the receptor protein or its other forms labeled with radioactive substance or enzyme is added, and the monoclonal antibody bound to the solid phase is detected.

Monoclonal antibody can be selected by publicly known methods or its modified methods. Usually, medium for animal cells supplemented with HAT (hypoxanthine, aminopterin, thymidine) is used. Any medium in which hybridoma can grow is used for selection and clonal growth. For example, RPMI 1640 medium containing 1 - 20%, preferably 10 - 20% fetal calf serum, GIT medium (Wako Pure Cheimcal Industries, Ltd.) containing 1 - 10% fetal calf serum, and serum-free medium for hybridoma culture (SFM-101, Nissui Pharmaceutical Co., Ltd.) can be used. The temperature for culture is usually 20 - 40°C, preferably about 37°C. The duration of culture is usually 5 days to 3 weeks, preferably 1 - 2 weeks. Usually, the cells can be cultured under 5% CO₂ gas. The antibody titer in hybridoma culture supernatant can be measured by the same procedure described for antibody titer in antiserum described above.

### (b) Purification of monoclonal antibody

The monoclonal antibodies can be purified by the immunoglobulin purification methods conventionally used for purification of polyclonal antibody [e.g. salting out, ancohol precipitation, isoelectric precipitation, electrophoresis, adsorption-desorption method using ion exchangers (e.g. DEAE), ultracentrifugation, gel filtration, specific purification method in which only antibody is collected using an active adsorbent such as antigen-bound solid phase, protein A, and protein G, and the antibody is obtained by dissociating the antibody from the binding].

### [Preparation of polyclonal antibody]

The polyclonal antibody of this invention can be manufactured by publicly known methods or its modified methods. For example, a complex of immunogen (antigen of the receptor protein or its other forms) and carrier protein is prepared, and mammals are immunized with this complex by the same method as described for preparation of monoclonal antibody, and the material containing the antibodies against the receptor protein or its other forms are collected and purified.

Regarding the complex of immnogen and carrier protein for immunizing mammals, for the type of carrier protein and the mixing ratio of carrier to the hapten, any carrier crosslinked to the hapten at any ratio can be used if the antibody is efficiently produced against the hapten immunized with crosslinked carrier. For example, bovine serum albumin, bovine thyroglobulin, or keyhole limpet hemocyanin is coupled to hapten at a weight ratio of carrier to hapten of about 0.1 - 20, preferably about 1 - 5.

Various condensation agents can be used for coupling of carrier to hapten. Active ester reagents containing glutaraldehyde, carbodiimide, maleimide active ester, thiol group, and dithiopyridyl group are used.

The condensation product is administerd with or without carrier and diluent to the site at which antibody can be produced in warm-blooded animals. To increase the antibody productivity, Freund complete or incomplete adjuvant may be administered when the condensation product is administered. Usually, the condensation product may be administered evry once per about 2 - 6 weeks, about 3 - 10 times in total.

The polyclonal antibody can be collected from the blood and ascites, preferably from the blood of mammals immunized by the method described above.

The polyclonal antibody titer in antiseurm can be measured by the same procedure for the serum antibody titer described above. The polyclonal antibody can be purified according to the same purification method for immunoglobulin as that of the monoclonal antibody described above.

The receptor protein and its salts, its partial peptides and its salts, and DNAs encoding said receptor protein or its partial peptides can be used for the following items: (1) Determination of ligands (agonist) for the G-protein-coupled receptor protein of this invention; (2) Prophylactic and/or therapeutic drugs for diseases related to dysfunction of the G-protein-coupled receptor protein of this invention; (3) Drugs for gene diagnosis; (4) Method for screening compounds that change the expression level of the receptor protein of this invention or its partial peptides; (5) Prophylactic and/or therapeutic drugs for various diseases containing a compound that changes the expression level of the receptor protein of this invention or its partial peptides; (6) Method for quantification of ligands for the G-protein-coupled receptor protein of this invention; (7) Method for screening compounds (agonists and antagonists) that change the binding property between the G-protein-coupled receptor protein of this invention and ligands; (8) Prophylactic and/or therapeutic drugs for various diseases containing a compound that changes the binding property between the G-protein-coupled receptor protein of this invention and ligands; (9) Quantification of the receptor protein of this invention, its partial peptides, and its salts; (10) Method for screening compounds that change the amount of the receptor protein of this invention or its partial peptides in cell membranes; (11) Prophylactic and/or therapeutic drugs for various diseases containing a compound that changes the amount of the receptor protein of this invention or its partial peptides in cell membranes; (12) Neutralization by antibodies against the receptor protein of this invention, its partial peptides, and its salts; and (13) Preparation of non-human animals that possess the DNA encoding the G-protein-coupled receptor protein of this invention.

Especially, using the receptor binding assay system using the expression system of the recombinant G-protein-coupled receptor protein of this invention, compounds (e.g. agonist and antagonist) that change the binding property of human- or mammals-specific ligands for the G-protein-coupled receptor protein can be screened, and the agonist and antagonist can be used as prophylactic/therapeutic drugs for various diseases.

Concrete explanations as to use of the receptor protein of this invention, its partial peptides, and its salts (receptor protein of this invention and its other forms), DNAs encoding the receptor protein of this invention and its partial peptides (DNA of this invention), and antibodies against the receptor protein of this invention and its other forms (antibody of this invention) are described below.

### (1) Determination of ligands (agonist) for the G-protein-coupled receptor protein of this inveniton

The receptor protein, its salts, its partial peptides, and its salts of this invention are useful as reagents for searching and determining ligands (agonist)for the receptor protein of this invention and its salts.

This invention provides a method for determining ligands for the receptor protein of this invention which is characterized by contacting test compounds to the receptor protein of this invention, its salts, its partial peptides, or its salts.

Test compounds include publicly known ligands (e.g. angiotensin, bombesin, canavinoid, cholecystokinin, glutamine, serotonin, melatonin, neuropeptide Y, opioid, purines, vasopressin, oxytocin, PACAP, secretin, glucagon, calcitonin, adrenomedulin, somatostatin, GHRH, CRF, ACTH, GRP, PTH, vasoactive intestinal and related polypeptide (VIP), somatostatin, dopamine, motilin, amylin, bradykinin, calcitonin gene-related peptide (CGRP), leukotrienes, pancreastatin, prostaglandins, thromboxane, adenosine, adrenaline, α and β-chemokines (e.g. IL-8, GROα, GROβ, GROγ, NAP-2, ENA-78, PF4, IP10, GCP-2, MCP-1, HC14, MCP-3, I-309, MIP1α, MIP-1β, RANTES), endothelin, entrogastrin, histamin, neurotensin, TRH, pancreatic polypeptide, and galanin) as well as other substances, for example, tissue extracts amd cell culture supernatants from humans and mammals (e.g. mice, rats, pigs, cattle, sheep, mnkeys). Said tissue extracts and cell culture supernatants, for example, are added to the receptor protein of this invention and fractionated by measuring the cell-stimulating activity, and finally, a single ligand can be obtained.

Concretely, in the method for determining the ligand of this invention using the receptor protein of this invention, its partial peptides, or its salts, or using the constructed expression system for recombinant receptor protein in the receptor binding assay, compounds (e.g. peptide, protein, non-peptide compound, synthetic compound, fermentation product) or its salts that bind to the receptor protein of this invention so that have the cell stimulating activity (e.g. activity that promotes or inhibits arachidonic acid release, acetylcholine release, intracellular Ca²⁺ release, intracellular cAMP production, intracellular cGMP production, inositol phosphate production, changes in cellmembrane potential, phosphorylation of intracellular proteins, activation of c-fos, and pH reduction) are determined.

The method for determining the ligand of this invention is characterized by, for example, measurement of the amount of the test compound bound to the receptor protein or its partial peptides and the cell-stimulating activity when the test compound is contacted to the receptor protein of this investigation or its partial peptides.

More concretely, this invention provides the methods for determining the ligand for the receptor protein or its salts of this invention characterized by follows..
① Method for determining ligands for the receptor protein or its salts of this invention, characterized by measuring the amount of labeled test compound bound to the receptor protein or its salts, or its partial peptides or its salts after contacting the labeled test compound to the receptor protein or its salts, or the partial peptides or its salts.
② Method for determining ligands for the receptor protein or its salts of this invention characterized by measuring the amount of labeled test compound bound to cells containing the receptor protein of this invetion or its membrane fraction after contacting the labeled test compound to said cells or said membrane fraction.
③ Method for determining ligands for the receptor protein of this invention characterized by measuring the amount of labeled test compound bound to said receptor protein or its salts, when labeled test compound is contacted to the receptor protein of this invention expressed on the cell membrane by culturing transformant containing the DNA encoding the receptor protein of this invention.
④ Method for determining ligands for the receptor protein or its salts of this invention characterized by measuring the receptor protein- mediated cell-stimulating activity (e.g. activity that promotes or inhibits arachidonic acid release, acetylcholine release, intracellular Ca²⁺ release, intracellular CAMP production, intracellular cGMP production, inositol phosphate production, changes in cell membrane potential, phosphorylation of intracellular proteins, activation of c-fos, and pH reduction), when test compound is contacted to cells containing the receptor protein of this invention; and
⑤ Method for determining ligands for the receptor protein or its salts of this invention and the receptor protein-mediated cell-stimulating activity (e.g. activity that promotes or inhibits arachidonic acid release, acetylcholine release, intracellular Ca²⁺ release, intracellular cAMP production, intracellular CGMP production, inositol phosphate production, changes in cellmembrane potential, phosphorylation of intracellular proteins, activation of c-fos, and pH reduction) when test compound is contacted to the receptor protein expressed on the cell membrane by culturing transformant containing DNA encoding the receptor protein of this invention.

It is preferable to perform the tests described in ④ and ⑤ after the tests described in ① to ③ are performed and the test compound is confirmed to bind to the receptor protein of this invention.

For the receptor protein used in the methods for determining the ligand, any molecule that contains the receptor protein of this invention or the partial peptides of this invention described above can be used, but the receptor protein expressed in a large scale using animal cells is appropriate.

For manufacture of the receptor protein of this invention, the expression methods described above are used, but it is preferable to express the DNA encoding the receptor protein in mammalian cells or insect cells. For the DNA fragment encoding the objetive protein region, the complementary DNA is usually used, but it is not restricted to the complementary DNA. For example, gene fragments and synthetic DNA may be used. To introduce DNA. fragment encoding the receptor protein of this invenion into host animal cells and efficiently express the DNA fragment, it is preferable to insert the DNA fragment to downstream of polyhedorin promoter of nuclear polyhedrosis virus (NPV) belonging to baculovirus which host is insect, SV40-derived promoter, retrovirus promoter, metallothionein promoter, human heat shock promoter, cytomegalovirus promoter, or SRα promoter. The amount and quality of the expressed receptor can be examined by publicly known methods. For example, it can be performed according to the method described in the literature [Nambi, P. et al., The Journal of Biological Chemistry (J. Biol. Chem.) Vol. 267, pp. 19555-19559, 1992].

Therefore, in the methods for determining ligand of this invention, the receptor protein, its partial peptides purified by known methods, or its salts may be used as the molecule containing the receptor protein of this invention or its partial peptides or its salts, and cells containing the receptor protein or its cell membrane fraction may also be used.

When cells containing the receptor protein of this invention are used in the methods for determining ligand of this invention, the cells may be fixed in glutaraldehyde or formalin. The cells can be fixed according to publicly known methods.

'The cells containing the receptor protein of this invention' means the host cells expressing the receptor protein of this invention, and for the host cells, *Eschericia coli*, *Bacillus subtilis*, yeast, insect cells, and animal cells are used.

The cell membrane fraction means the fraction containing abundant cell membranes obtained by publicly known methods after disruption of the cells. The methods for cell disruption include crushing the cells using a Potter-Elvehjem homogenizer, disruption using a Waring blender or polytron (Kinematica Co.), disruption by ultrasonication, and disruption by passing the cells through a narrow nozzle with compressing the cells using a French Press. For the cell membrane fractionation, fractionation based on centrifugal force such as fractionation by centrifugation and density gradient centrifugation are mainly used. For example, disrupted cell suspension is centrifuged at a low speed (500 - 3,000 rpm) for a short time (usually about 1 - 10 minutes), and the supernatant is centrifuged at a high speed (15,000 - 30,000 rpm) for usually 30 minutes - two hours, and the obtained precipitate is used as the membrane fraction. Said membrane fraction contains many membrane components such as the expressed receptor protein and phospholipids and membrane proteins derived from the cells.

For the amount of the receptor protein contained in the cells containing the receptor protein or the membrane fraction, 10³ - 10⁸ molecules per cell is preferred, and 10⁵ - 10⁷ molecules per cell is appropriate. As the expression level increases, the ligand-binding activity (specific activity) of the membrane fraction increases, which allows not only construction of a highly sensitive acreening system but also measurement of a large number of samples using the same lot.

To perform the tests in ① to ③ described above for determinig ligands for the receptor protein of this invention or its salts, appropriate receptor protein fraction and labeled test compounds are necessary.

For the receptor protein fraction, natural receptor protein fraction or recombinant receptor fraction having equivalent activity to that of the natural receptor protein fraction is preferred. The equivalent activity means equivalent ligand-binding activity and signal tranduction activity.

For the labeled test compounds, [³H]-, [¹²⁵I]-, [¹⁴C]-, or [³⁵S]-labeled angiotensin, bombesin, canavinoid, cholecystokinin, glutamine, serotonin, melatonin, neuropeptide Y, opioid, purines, vasopressin, oxytocin, PACAP, secretin, glucagon, calcitnonin, adrenomedulin, somatostatin, GHRH, CRF, ACTH, GRP, PTH, vasoactive intestinal and related polypeptide (VIP), somatostatin, dopamine, motilin, amyrin, bradykinin, calcitonin gene-related peptide (CGRP), leukotriens, pancreastatin, prostaglandins, thromboxane, adenosine, adrenaline, α and β-chemokines (e.g. IL-8, GROα, GROβ, GROγ, NAP-2, ENA-78, PF4, IP10, GCP-2, MCP-1, HC14, MCP-3, I-309, MIP1α, MIP-1β, and RANTES), endothelin, enterogastrin, histamine, neurotensin, TRH, pancreatic polypeptide, or galanin are appropriate.

Concretely, to perform the method for determining ligands for the receptor protein of this invention or its salts, firstly, by suspending the cells or cell emmebrane fraction containing the receptor protein of this invention into buffer appropriate for the determination, the receptor preparation is prepared. For the buffer, any buffer that does not inhibit the binding of ligands to the receptor protein such as phosphate buffer and Tris-hydrochloride buffer pH 4 - 10 (preferably pH 6 - 8) can be used. To reduce non-specific binding, a surfactant such as CHAPS, Tween-80™ (Kao-Atras Co.), digitonin, and deoxycholate and various proteins such as bovine serum albumin and gelatin may be added to the buffer. To inhibit degradation of the receptor and ligands by proteases, protease inhibitors such as PMSF, leupeptin, E-64 (Peptide Research Laboratory, Co.), and pepstatin may be added. To 0.01 - 10 ml of the receptor solution, a specified amount (5,000 - 500,000 cpm) of [³H] -, [¹²⁵I]-, [¹⁴C]-, or [³⁵S]-labeled test compound is added. To examine the non-specific binding (NSB), reaction tubes containing a highly excessive amount of non-labeled test compound are prepared. The reaction is performed at about 0°C - 50°C, preferably about 4°C - 37°C, for about 20 minutes - 24 hours, preferably for about 30 minutes - 3 hours. After the reaction, the reaction solution is filtered through a glass fiber filter and the filter was washed with an appropriate amount of the buffer. The radioactivity remaining on the glass fiber filter is measured using a liquid scintillation counter or γ - counter. When the count obtained by subtracting non-specific binding (NSB) from the total binding (B) exceeds 0 cpm, the test compound canbe selcted as a ligand (agonist) for the receptor protein of this invention or its salts.

To perform the methods for demtermining ligands for the receptor protein of this invention or its salts described in ④ and ⑤ , the receptor protein mediated cell-stimulating activity (e.g. activity that promotes or inhibits arachidonic acid release, acetylcholine release, intracellular Ca²⁺ release, intracellular cAMP production, intracellular cGMP production, inositol phosphate production, changes in cellmembrane potential, phosphorylation of intracellular proteins, activation of c-fos, and pH reduction) can be measured using publicly known methods or commercial kits. Concretely, fisrt, cells containing the receptor protein are cultured in multiwell plates. Before determination of ligands, the medium is exchanged to fresh medium or appropriate buffer that exhibits no toxicity for the cells. After the cells are incubated with test compound for a specified time, the cells are extracted or the supernatant is collected, and the product is quantified according to the corresponding method. When the production of indicator substance for the cell-stimulating activity (e.g. arachdonic acid) is difficult to examine due to degrading enzymes contained in the cells, inhibitors of the degrading enzymes may be added to the assay system. For detection of the inhibitory activity on cAMP production, the baseline production in the cells is increased by forskolin, and the inhibitory effect on the increased baseline production is detected.

The kits for determining ligands for the receptor protein of this invention or its salts contain the receptor protein or its salts, the partial peptides of this invention or its salts, cells containing the receptor protein of this invention, or the membrane fraction of cells containing the receptor protein of this invention.

Examples of the kit for determining ligands of this invenion are as follow.
1. Reagents for ligand determination
   ① Buffers for measurement and washing
      Hanks' Balanced Salt Solution (Gibco Co.) supplemeted with 0.05% bovine serum albumin (Sigma Co.).
      The solution is sterilized by filteration through a 0.45 µm filter, and stored at 4°C or may be prepared at use.
   ② Standard G-protein-coupled receptor protein
      CHO cells expressing the receptor protein of this invention are passaged in 12-well plates at a density of 5 x 10⁵ cells/well and cultured at 37°C under 5% CO₂ and 95% air for two days.
   ③ Labeled test compounds
      Commercial [³H] -, [¹²⁵I] -, [¹⁴C] -, or [³⁵S]-labeled compounds or compounds labeled by appropriate methods.
      Aqueous solutions of the compounds are stored at 4°C or -20°C, and the solution is diluted to 1 µM with measurment buffer at use. Test compounds which are difficult to solve into water are dissolved in dimethylformamide, DMSO, or methanol.
   ④ Non-labeled compounds
      Non-labeled forms of the labeled compounds are prepared at a concentration of 100 to 1,000-fold higher than that of the labeled compound.
2. Measurement methods
   ① CHO cells expressing the receptor protein of this invention are cultured in 12-well culture plates and washed twice with 1 ml of measurement buffer, and 490 µl of the measurement buffer is added to each well.
   ② Five µl of labeled test compound is added, and the cell are incubated at room temperature for one hour. To measure the non-specific binding, 5 µl of the non-labeled compound is added.
   ③ The reaction solution is removed, and the wells are washed three times with washing buffer. The labeled test compound bound to the cells is dissolved with 0.2N NaOH-1% SDS, and mixed with 4 ml of liquid scintillator A (Wako Pure Chemical Industries, Ltd.)
   ④ The radioactivity is measured using a liquid scintillation counter (Beckman Co.)

The ligands that bind to the receptor protein of this invention or its salts include substances specifically present in the brain, pituitary gland, and pancreas. For example, angiotensin, bombesin, canavinoid, cholecystokinin, glutamine, serotonin, melatonin, neuropeptide Y, opioids, purine, vasopressin, oxytocin, PACAP, secretin, glucagon, calcitnonin, adrenomedulin, somatostatin, GHRH, CRF, ACTH, GRP, PTH, vasoactive intestinal and related polypeptide (VIP), somatostatin, dopamine, motilin, amyrin, bradykinin, calcitonin gene-related peptide (CGRP), leukotriens, pancreastatin, prostaglandins, thromboxane, adenosine, adrenaline, α and β-chemokines (e.g. IL-8, GROα, GROβ, GROγ, NAP-2, ENA-78, PF4, IP10, GCP-2, MCP-1, HC14, MCP-3, I-309, MIP1α, MIP-1β, and RANTES), endothelin, enterogastrin, histamine, neurotensin, TRH, pancreatic polypeptide, and galanin are used.

### (2) Prophylactic and/or therapeutic drugs for diseases related to dysfunction of the G-protein-coupled receptor protein of this invention

When a compound is clarified to be a ligand of the receptor protein of this invention by the methods described in (1), ① the receptor protein of this invention, or ② the DNA encoding the receptor protein can be used, depending on the action of the ligand, as prophylactic and/or therapeutic drug for diseases related to dysfunction of the receptor protein of this invention.

For example, when the physiological action of the ligand cannot be expected due to reduction of the receptor protein of this invention in a patient (deficiency of the receptor protein), the action of the ligand can be exhibited by the according to methods: ① the receptor protein of this invention is administered to the patient to supplement the amount of the receptor protein; or ② the amount of the receptor protein is increased in the patient by: i) administeration of the DNA encodning the the receptor protein of this invention to express in the patient; or ii) introduction and expression of the DNA encodning the the receptor protein of this invention in the objective cells, then the cells are transplanted in the patient. Therefore, the DNA encoding the receptor protein of this invention is useful as a safe and low toxic prophylactic and/or therapeutic drug for diseases related to dysfuntion of the receptor protein of this invention.

The receptor protein of this invention have about 30% homology to MAS, which is one of G-protein-coupled receptor proteins, at the amino acid sequence level. Since it was reported that changes in the central function such as increased anxiety are observed in MAS gene decifient mice [J. B. C., 273 (No. 19), 11867-11873 (1998)], MAS gene is considered to have some action on the expression of central function. Therefore, the receptor protein of this invenion, which has homology to MAS, is useful for prophylaxis and/or therapy for diseases related to central dysfunction (e.g. psychosis including anxiety, schizophrenia, manic-depressive psychosis, dementia, mental retardation, and dyskinesia). Since it was reported that MAS gene is highly expressed in various peripheral organs immediately after birth in rats, and after maturation, among the organs other than the center, a high expression is observed in the testes [FEBS Lett. 357: 27-32 (1995)], MAS gene is considered to play an important role in cell growth, acquisition of function, and reproduction. Therefore, the receptor protein of this invenion, which has homology to MAS, is useful for prophylaxis and/or therapy for diseases of the respiratory organs, circulatory organs, digenstive tract, liver, gallbladder, and pancreas and endocrine diseases.

When the receptor protein of this invention is used as the prophylactic and/or therapeutic drug as described above, the preparation can be obtained according to the conventional methods.

When the DNA encoding the receptor protein of this invention (DNA of this invention) is used as the prophylactic and/or therapeutic drug as described above, the DNA of this invention alone or inserted into an appropriate vector such as retrovirus vector, adenovirus vector, and adenovirus-associated virus vector can be used according to the conventional means. The DNA of this invention is administered with or without an adjuvant for facilitating the uptake, using a catherter such as genetic gun and hydrogel catherter.

For example: 1) the receptor protein of this invention; or 2) the DNA encoding the receptor protein can be used orally as a sugar coated tablet, capsule, elixir, and microcapsule, or non-orally as injection such as aseptic solution in water or other pharmaceutically accceptable liquid or suspension. For example, preparation of: 1) the receptor protein of this invention; or 2) the DNA encoding the receptor protein can be manufactured by mixing with physiologically acceptable publicly known carrier, flavor, excipient, vehicle, antiseptic, stabilizer, and binder in a unit-dosage form required for generally approved drug preparation. The amount of the active ingredient in these preparations is set to an appropriate volume within the specified range.

For the additive that can be mixed in tablets and capsules, for example, binders such as gelatin, cornstarch, tragacanth, and acacia, excipients such as crystalline cellulose, imbibers such as cornstarch, gelatin, and alginic acid, lubricants such as magnesium stearate, sweeteners such as sucrose, lactose and saccharin, and flavors such as peppermint, akamono oil and cherry are used. When the unit-dosage form is capsule, liquid carrier such as fat and oil can be contained. Aseptic compositions for injection can be formulated according to the usual preparation procedure such as dissolving or suspending the active substance in vehicle, e.g. water for injection, and natural plant oils e.g. sesame oil and coconut oil. For the aqueous solution for injection, for example, physiological saline and isotonic solutions (e.g. D-sorbitol, D-mannitol, sodium hydrochloride) containing glucose and other adjuvant are used. Appropriate dissolution-assisting agents, for example, alcohol (e.g. ethanol), polyalcohol (e.g. propylene glycol, polyethylene glycol), nonionic surfactant (e.g. polysorbate 80™, HCO-50) may be combined. For the oily solution, for example, sesame oil and soybean oil are used, and dissolution-assisting agents such as benzyl benzoate and benzyl.alcohol may be combined.

The prophylactic/therapeutic drugs described above may be combined with, for example, buffers (e.g. phosphate buffer solution, sodium acetate buffer solution), analgesics (e.g. benzalkonium chloride, procaine hydrochloride), stabilizers (e.g. human serum albumin, polyethylene glycol), preservatives (e.g. benzylalcohol, phenol), and antioxidants. The preparated solution for injection is usually filled in appropriate ampoules.

Since the preparations obtained as described above are safe and low toxic, they can be administered to, for example, humans and mammals (e.g. rats, rabbits, sheep, pigs, cattle, cats, dogs, monkeys).

The dosage of the receptor protein of this invention differs depending on the target individuals, target organs, symptoms and administration method. When it is administered orally, in general, for schizophrenic patients (60 kg body weight), for example, about 0.1 mg - 100 mg per day, preferably about 1.0 mg - 50 mg per day, more preferably about 1.0 mg - 20 mg per day is administered. When it is administered non-orally, the dosage per dosing differs depending on the target individual, target organ, symptom, and administration method. For example, in case of injection, to schizophrenic patients (60 kg body weight), for example, it is desirable to intravenously inject about 0.01 - 30 mg per day, preferably about 0.1 - 20 mg per day, more preferably about 0.1 - 10 mg per day. Converting the dosage for 60 kg, the protein can be administered to other animals.

The dosage of the DNA of this invention differs depending on the target individual, target organ, symptom, and administration method. When it is administered orally, in general, for schizophrenic patients (60 kg body weight), for example, about 0.1 mg - 100 mg per day, preferably about 1.0 mg - 50 mg per day, more preferably about 1.0 mg - 20 mg per day is administered. When it is administered non-orally, the dosage per dosing differs depending on the target individual, target organ, symptom, and administration method. In case of injection, for schizophrenic patients (60 kg body weight), for example, usually it is desirable to intravenously inject about 0.01 - 30 mg per day, preferably about 0.1 - 20 mg per day, more preferably about 0.1 - 10 mg per day. Converting the dosage for 60 kg, the protein can be administered to other animals.

### (3) Gene diagnostic agent

Since, using the DNA of this invention as a probe, abnormalities (gene aberration) in the DNA and mRNA encoding the receptor protein or its partial peptides of this invention can be detected in humans and mammals (e.g. rats, rabbits, sheep, pigs, cattle, cats, dogs, monkeys), the DNA is useful as gene diagnostic agents for, for example, injuries, mutations, and reduction of expression of the DNA and mRNA, and an increase and overexperssion of the DNA and mRNA.

The gene diagnosis using the DNA of this invention described above can be performed by publicly known methods, for example, northern hybridization and PCR-SSCP (Genomics Vol. 5, 874-879 (1989); Proc. Natl. Acad. Sci. USA Vol. 86, 2766-2770 (1989)).

### (4) Methods for screening compounds that change the expression level of the receptor protein or its partial peptides of this invention

Using as a probe, the DNA of this invention can be used for screening of compounds that change the expression level of the receptor protein or its partial peptides of this invention.

This invention provides methods for screening compounds that change the expression level of the receptor protein or its partial peptides of this invention by measuring the amount of mRNA of the receptor protein or its partial peptides of this invention contained in, for example, (i) ① blood, ② specific organs, and ③ tissues or cells isolated from the organs of non-human mammals and (ii) transformants.

Concretely, the amount of mRNA of the receptor protein and its partial peptides of this invention are measured as follows.
(i) Normal or disease models of non-human animals (e.g. mice, rats, rabbits, sheep, pigs, cattle, cats, dogs, monkeys, more specifically, dementia rats, obese mice, arteriosclerotic rabbits, tumor-bearing mice) receive administration of a drug (e.g. nootroopic drugs, hypotensive drugs, anticancer drugs, antiobesitic drugs) or physical stress (e.g. soaking stress, electric stress, light and darkness, low temperature) are provided, and the blood, specific organs (e.g. brain, liver, kidneys), or tissue or cells isolated from the organs are obtained after a specified time.

The mRNA of the receptor protein or its partial peptides of this invention is extracted, for example, from the cells by the conventional method and quantified using, for example, TaqManPCR, and can be analyzed by northern blot using publicly known methods. (ii) Transformants expressing the receptor protein or its partial peptides of this invention are prepared according to the methods described above, and the mRNA of the receptor protein or its partial peptides of this invention can be quantified and analyzed as described above.

Compounds that change the expression level of the receptor protein or its partial peptides of this invention can be screened by the following procedures.
(i) To normal or disease models of non-human mammals, test compound is administered at a specified time before (30 minutes - 24 hours before, preferably 30 minutes - 12 hours before, more preferably 1 hour - 6 hours before), at a specified time after (30 minutes - 3 days after, preferably 1 hour - 2 days after, more preferably 1 hour - 24 hours after), or simultaneously with a drug or physical stress. At a specified time (30 minute - 3 days, preferably 1 hour - 2 days, more preferably 1 hour - 24 hours) after administration of the test compound, the mRNA level of the receptor protein or its partial peptides of this invention contained in cells are quantified and analyzed.
(ii) When transformants are cultured by the conventional method, a test compound is mixed in the culture medium. After a specified time (after 1 day - 7 days, preferably after 1 day - 3 days, more preferably after 2 days - 3 days), the mRNA level of the receptor protein or its partial peptides of this invention contained in the transformant can be quantified and analyzed.

Compounds and its salts that can be obtained by the screening methods for this invention are compounds having function that change the expression level of the receptor protein or its partial peptides of this invention. They are concretely; (a) compounds that potentiate the cell-stimulating activity mediated by the G-protein-coupled receptor (e.g. activity that promotes or inhibits arachidonic acid release, acetylcholine release, intracellular Ca²⁺ release, intracellular cAMP production, intracellular cGMP production, inositol phosphate production, changes in cell membrane potential, phosphorylation of intracellular proteins, activation of c-fos, and pH reduction) by increasing the expression level of the receptor protein or its partial peptides; and (b) compounds that decrease the cell-stimulating activity by reducing the expression level of the receptor protein or its partial peptides of this invention.

The compounds include peptides, proteins, non-peptide compounds, synthetic compounds, and fermentation products, and may be novel or publicly known compounds.

Compounds that potentiate the cell-stimulating activities are useful as safe and low-toxic pharmaceuticals for potentiation of the physiological activity of the receptor protein or its other forms of this invention.

Compounds that decrease the cell-stimulating activities are useful as safe and low-toxic pharmaceuticals for reduction of the physiological activity of the receptor protein or its other forms of this invention.

When compounds or its salts obtained by the screening methods for this invention are used as a pharmaceutical component, the compound can be formulated by the conventional methods. For example, as described for the pharmaceuticals containing the receptor protein of this invention, the compounds can be prepared as tablets, capsules, elixir, microcapsules, aseptic solution, or suspension.

Since the preparations obtained as described above are safe and low-toxic, they can be administered to humans and mammals (e.g. rats, rabbits, sheep, pigs, cattle, cats, dogs, monkeys).

The dosage of the compounds or its salts differs depending on the target individual, target organ, symptom, and administration method. When it is administered orally, in general, for schizophrenic patients (60 kg body weight), for example, about 0.1 mg - 100 mg per day, preferably about 1.0 mg - 50 mg per day, more preferably about 1.0 mg - 20 mg per day is administered. When it is administered non-orally, the dosage per dosing differs depending on the target individual, target organ, symptom, and administration method. In case of injection, for schizophrenic patients (60 kg body weight), for example, usually it is desirable to intravenously inject about 0.01 - 30 mg per day, preferably about 0.1 - 20 mg per day, more preferably about 0.1 - 10 mg per day. Converting the dosage for 60 kg, the protein can be administered to other animals.

### (5) Prophylactic and/or therapeutic drugs for various diseases containing compounds that change the expression level of the receptor protein or its partial peptides of this invention

As described above, the receptor protein of this invention is considered to play some important role in vivo, for example, a role in the central function. Therefore, compounds that change the expression level of the receptor protein and its partial peptides of this invention can be used as prophylactic and/or therapeutic drugs for diseases related to dysfunction of the receptor protein of this invention.

When the compounds are used as prophylactic and/or therapeutic drugs for diseases related to dysfunction of the receptor protein of this invention, the preparations can be obtained according to the conventional methods.

For example, the compounds can be administered orally as a sugar coated tablet, capsule, elixiria, and microcapsule, or non-orally as injection such as aseptic solution or suspension in water or other pharmaceutically acceptable liquid. For example, preparations of the compounds can be manufactured by mixing with physiologically acceptable publicly known carrier, flavor, excipient, vehicle, antiseptic, stabilizer, and binder in a unit-dosage form required for generally approved drug preparation. The amount of the active ingredient in these preparations is set to an appropriate volume within the specified range.

For the additive that can be mixed in tablets and capsules, for example, binders such as gelatin, cornstarch, tragacanth, and acacia, excipients such as crystalline cellulose, imbibers such as cornstarch, gelatin, and alginic acid, lubricants such as magnesium stearate, sweeteners such as sucrose, lactose and saccharin, and flavors such as peppermint, akamono oil and cherry are used. When the dosage form is a capsule, liquid carrier such as fat and oil can be contained. Aseptic compositions for injection can be formulated according to the usual preparation procedure such as dissolving or suspending the active substance in vehicle, e.g. water for injection, and natural plant oils e.g. sesame oil and coconut oil. For the aqueous solution for injection, for example, physiological saline and isotonic solutions (e.g. D-sorbitol, D-mannitol, sodium hydrochloride) containing glucose and other adjuvant are used. Appropriate dissolution-assisting agents, for example, alcohol (e.g. ethanol), polyalcohol (e.g. propylene glycol, polyethylene glycol), nonionic surfactant (e.g. polysorbate 80™, HCO-50) may be combined. For the oily solution, for example, sesame oil and soybean oil are used, and dissolution-assisting agents such as benzyl benzoate and benzyl alcohol may be combined.

The prophylactic/therapeutic drugs described above may be combined with buffers (e.g. phosphate buffer solution, sodium acetate buffer solution), analgesics (e.g. benzalkonium chloride, procaine hydrochloride), stabilizers (e.g. human serum albumin, polyethylene glycol), preservatives (e.g. benzylalcohol, phenol), and antioxidants. The preparated solution for injection is usually filled in appropriate ampoules.

Since the preparations obtained as described above are safe and low-toxic, they can be administered to, for example, humans and mammals (e.g. rats, rabbits, sheep, pigs, cattle, cats, dogs, monkeys).

The dosage of the compounds or its salts differs depending on the target individual, target organ, symptom, and administration method. When it is administered orally, in general, for schizophrenic patients (60 kg body weight), for example, about 0.1 mg - 100 mg per day, preferably about 1.0 mg - 50 mg per day, more preferably about 1.0 mg - 20 mg per day is administered. When it is administered non-orally, the dosage per dosing differs depending on the target individual, target organ, symptom, and administration method. In case of injection, for schizophrenic patients (60 kg body weight), for example, it is desirable to intravenously inject about 0.01 - 30 mg per day, preferably about 0.1 - 20 mg per day, more preferably about 0.1 - 10 mg per day. Converting the dosage for 60 kg, the protein can be administered to other animals.

### (6) Methods for quantifying ligands for the G-protein-coupled protein of this invention

Since the receptor protein and its other forms of this invention have ligand-binding activity, the ligand concentration in vivo can be quantified with good sensitivity.

The quantification methods for this invention can be used in combination with, for example, competitive method. The ligand concentration in a test sample can be measured by contacting the test sample to the receptor protein or its other forms of this invention. Concretely, the methods can be used by following, for example, the methods described in ① or ② below or its modified methods.
① Irie, H. ed. 'Radioimmunoassay,' Kodansha (1974)
② Irie, H. ed. 'Sequel to the Radioimmunoassay,' Kodansha (1979)

### (7) Methods for screening compounds (agonist, antagonist) that change the binding property between the G-protein-coupled receptor protein of this invention and ligands

Using the receptor protein or its other forms of this invention, or using receptor binding assay system consisting of the expression system of the recombinant receptor protein or its other forms of this invention, compounds (e.g. peptides, proteins, non-peptide compounds, synthetic compounds, fermentation products) and its salts that change the binding property between ligands and the receptor protein or its other forms can be efficiently screened.

Such compounds include (a) compounds that have the G-protein-coupled receptor-mediated cell-stimulating activity (e.g. activity that promotes or inhibits arachidonic acid release, acetylcholine release, intracellular Ca²⁺ release, intracellular cAMP production, intracellular cGMP production, inositol phosphate production, changes in cell membrane potential, phosphorylation of intracellular proteins, activation of c-fos, and pH reduction) (i.e. agonists of the receptor protein of this invention); (b) compounds that do not have the cell-stimulating activity (i.e. antagonists of the receptor protein of this invention); (c) compounds that potentiate the binding affinity between ligands and the G-protein-coupled receptor protein of this invention; or (d) compounds that reduce the binding affinity between ligands and the G-protein-coupled receptor protein of this invention (it is preferred to screen the compounds described in (a) using the methods for determining ligand described above).

Therefore, this invention provides methods for screening compounds or its salts that change the binding property between ligands and the receptor protein or its partial peptides or its salts, in which
(i) contact between the receptor protein or its partial peptides or its salts and a ligand is compared with
(ii) contact among the receptor protein or its partial peptides or its salts, test compound, and the ligand.

The screening methods for this invention are characterized by measuring and comparing, for example, the amount of ligand bound to the receptor protein or its other forms or the cell-stimulating activity between (i) and (ii).

More concretely, this invention provides the following screening methods.
① Method for screening compounds or its salts that change the binding property between ligands and the receptor protein or its other forms of this invention in which the amount of a labeled ligand bound to the receptor protein or its other forms is measured after contacting the labeled ligand to the receptor protein or its other forms of this invention and compared with that after contacting the labeled ligand and test compound to the receptor protein or its other forms of this invention.
② Method for screening compounds or its salts that change the binding property between ligands and the receptor protein or its other forms of this invention in which the amount of a labeled ligand bound to cells containing the receptor protein or its other forms of this invention or the membrane fraction of the cells is measured after contacting the labeled ligand to the cells or its membrane fraction, and compared with that after contacting the labeled ligand and test compound to the cells or its membrane fraction containing the receptor protein or its other forms of this invention.
③ Method for screening compounds or its salts that change the binding property between ligands and the receptor protein or its other forms of this invention in which a labeled ligand is contacted to the receptor protein or its other forms of this invention expressed on the cell membrane by culturing transformant containing the DNA encoding the receptor protein of this invention, and the amount of the labeled ligand bound to the receptor protein or its other forms is measured and compared with that after contacting the labeled ligand and a test compound to the receptor protein or its other forms of this invention expressed on the cell membrane by culturing transformant containing the DNA encoding the receptor protein of this invention.
④ Method for screening compounds or its salts that change the binding property between ligands and the receptor protein or its other forms of this invention in which compound that activates the receptor protein or its other forms of this invention (e.g. ligands for the receptor protein or its other forms) is contacted to cells containing the receptor protein or its other forms of this invention, and the receptor protein-mediated cell-stimulating activity (e.g. activity that promotes or inhibits arachidonic acid release, acetylcholine release, intracellular Ca²⁺ release, intracellular cAMP production, intracellular cGMP production, inositol phosphate production, changes in cell membrane potential, phosphorylation of intracellular proteins, activation of c-fos, and pH reduction) is measured and compared with that after contacting the compound that activates the receptor protein or its other forms of this invention and test compound to the cells containing the receptor protein or its other forms of this invention; and
⑤ Method for screening compounds and its salts that change the binding property between ligands and the receptor protein or its other forms of this invention in which compound that activates the receptor protein or its other forms of this invention (e.g. ligands for the receptor protein or its other forms) is contacted to the receptor protein or its other forms expressed on the cell membrane by culturing transformant containing the DNA of this invention, and the receptor protein-mediated cell-stimulating activity (e.g. activity that promotes or inhibits arachidonic acid release, acetylcholine release, intracellular Ca²⁺ release, intracellular CAMP production, intracellular cGMP production, inositol phosphate production, changes in cell membrane potential, phosphorylation of intracellular proteins, activation of c-fos, and pH reduction) is measured and compared with that after contacting the compound that activates the receptor protein or its other forms of this invention and test compound to the receptor protein or its other forms expressed on the cell membrane by culturing transformant containing the DNA of this invention.

Before the receptor protein and its other forms of this invention were obtained, for screening G-protein-coupled receptor agonists and antagonists, first, candidate compounds were obtained using cells or tissues or the cell membrane fraction from rats or other animals containing the G-protein-coupled receptor protein (primary screening), and then, it was necessary for the candidate compounds to be examined whether the compounds actually inhibit the binding of ligands to human G-protein-coupled receptor protein (secondary screening). When cells, tissues, or the cell membrane fraction were directly used, other receptor proteins were intermixed, and it was difficult to screen agonists and antagonists for the objective receptor protein.

However, for example, using the rat-derived receptor protein of this invention, the primary screening becomes unnecessary, and compounds that inhibit the binding of ligands to the G-protein-coupled receptor protein can be efficiently screened. Furthermore, it is easy to evaluate whether the obtained compound is agonist or antagonist.

Concrete explanation of the screening methods for this invention is described below.

First, for the receptor protein and its other forms of this invention used for the screening methods for this invention, any substance containing the receptor protein or its other forms of this invention described above may be used, and the cell membrane fraction of the organs from mammals containing the receptor protein or its other forms of this invention is preferred. However, it is very difficult to obtain human organs, and thus, it is preferable to use the rat-derived receptor protein or its other forms produced by large-scale expression in the recombinant.

To manufacture the receptor protein and its other forms of this invention, the methods described above are used, and it is preferable to manufacture them by expressing the DNA of this invention in mammalian and insect cells. For the DNA fragment encoding the objective protein region, the complementary DNA is used, but it is not restricted to the complementary DNA. For example, the gene fragments and synthetic DNA may be used. To introduce a DNA fragment encoding the receptor protein of this invention into host animal cells and efficiently express the DNA, it is preferable to insert the DNA fragment into downstream of polyhedorin promoter of nuclear polyhedrosis virus (NPV) belonging to baculovirus hosted by insects, SV40-derived promoter, retrovirus promoter, metallothionein promoter, human heat shock promoter, cytomegalovirus promoter, or SR α promoter. The amount and quality of the expressed receptor are examined by publicly known methods, for example, the method described in the literature [Nambi, P. et al., The Journal of Biological Chemistry (J. Biol. Chem.) Vol. 267; 19555-19559, 1992].

Therefore, in the screening methods for this invention, the substance that contains the receptor protein or its other forms of this invention may be the receptor protein or its other forms purified by publicly known methods, cells containing the receptor protein or its other forms, or the cell membrane fraction containing the receptor protein or its other forms.

In the screening methods for this invention, when cells expressing the receptor protein or its other forms of this invention are used, the cells may be fixed with gultaraldehyde or formalin. The cells can be fixed according to publicly known methods.

The cells containing the receptor protein or its other forms in this invention are host cells expressing the receptor protein or its other forms. For the host cells, *Escherichia coli, Bacillus subtilis,* yeast, insect cells, and animal cells are preferred.

Cell membrane fraction is a fraction containing abundant cell membranes obtained after disruption of the cells by publicly known methods. The cell disruption methods include crushing the cells using a Potter-Elvehjem homogenizer, crushing using a Waring blender or polytron (Kinematica Co.), disruption by ultrasonication, and disruption by passing the cells through a narrow nozzle with compressing the cells using a French Press. For the cell membrane fractionation, fractionation based on centrifugal force such as centrifugation for fractionation and density gradient centrifugation are mainly used. For example, disrupted cell suspension is centrifuged at a low speed (500 - 3,000 rpm) for a short time (usually about 1 - 10 minutes), then the supernatant is centrifuged at a high speed (15,000 - 30,000 rpm) for usually 30 minutes - 2 hours, and the obtained precipitate is used as the membrane fraction. The membrane fraction contains many membrane components such as the expressed receptor protein or its other forms, and phospholipids and membrane proteins derived from the cells.

For the amount of the receptor protein or its other forms contained in the cells or the membrane fraction, 10³ - 10⁸ molecules per cell is preferred, and 10⁵ - 10⁷ molecules per cell is appropriate. As the expression level increases, the ligand-binding activity (specific activity) of the membrane fraction increases, which allows not only construction of a highly sensitive screening system but also measurement of a large number of samples using the same lot.

To screen compounds that change the binding property between ligands and the receptor protein or its other forms of this invention described in ① to ③ , for example, appropriate receptor protein fraction and labeled ligand are necessary.

For the receptor protein fraction, native receptor protein fraction or recombinant receptor protein fraction with activity equivalent to that of the native receptor protein fraction is preferred. The equivalent activity means equivalent ligand-binding activity and signal transduction activity.

For the labeled ligand, labeled ligands and labeled ligand analogues are used. For example, [³H] - , [¹²⁵I] -, [¹⁴C]-, and [³⁵S]-labeled ligands are used.

Concretely, to screen compounds that change the binding property between ligands and the receptor protein or its other forms of this invention, first, the receptor protein standard is prepared by suspending cells or cell membrane fraction containing the receptor protein or its other forms of this invention in a buffer appropriate for screening. For the buffer, any buffer that does not inhibit the binding of ligands to the receptor protein such as phosphate buffer and Tris-hydrochloride buffer pH 4 - 10 (preferably pH 6 - 8) can be used. To reduce non-specific binding, a surfactant such as CHAPS, Tween-80™ (Kao-Atras Co.), digitonin, and deoxycholate may be added to the buffer. To inhibit degradation of the receptor and ligands by proteases, protease inhibitors such as PMSF, leupeptin, E-64 (Peptide Research Laboratory, Co.), and pepstatin may be added. To 0.01 - 10 ml of the receptor solution, a specified amount (5,000 - 500,000 cpm) of labeled ligand is added, and 10⁻⁴ M - 10⁻¹⁰ M test compound is simultaneously added. To examine the non-specific binding (NSB), reaction tubes containing a highly excessive amount of the non-labeled ligand are prepared. The reaction is performed at about 0°C - 50°C, preferably about 4°C - 37°C, for about 20 minutes - 24 hours, preferably for about 30 minutes - 3 hours. After the reaction, the reaction solution is filtrated through a glass fiber filter and the filter was washed with an appropriate volume of the buffer. The radioactivity remaining on the glass fiber filter is measured using a liquid scintillation counter or *γ*-counter. Regarding the count obtained by subtracting the amount of non-specific binding (NSB) from the count obtained in the absence of competitive substance (B₀) as 100%, when the amount of specific binding (B-NSB) is, for example, 50% or less, the test compound can be selected as a candidate substance having a potential of competitive inhibition.

To perform the methods for screening compounds that change the binding property between ligands and the receptor protein or its other forms of this invention described above in ④ and ⑤ , the cell-stimulating activity via the receptor protein (e.g. activity that promotes or inhibits arachidonic acid release, acetylcholine release, intracellular Ca²⁺ release, intracellular cAMP production, intracellular cGMP production, inositol phosphate production, changes in cell membrane potential, phosphorylation of intracellular proteins, activation of c-fos, and pH reduction) can be measured using publicly known methods or commercial kits.

Concretely, first, cells containing the receptor protein or its other forms of this invention are cultured in multiwell plates. Before the screening, the medium is exchanged to fresh medium or appropriate buffer that exhibits no toxicity for the cells. After the cells are incubated with test compound for a specified time, the cells are extracted or the supernatant is collected, and the product is quantified according to the corresponding method. When the production of indicator substance for the cell-stimulating activity (e.g. arachidonic acid) is difficult to examine due to degrading enzymes contained in the cells, inhibitors of the degrading enzymes may be added to the assay. For detection of the inhibitory activity on cAMP production, the baseline production in the cells is increased by forskolin, and the inhibitory effect on the increased baseline production is detected.

To screen by measuring the cell-stimulating activity cells expressing appropriate receptor protein are required. For the cells expressing the receptor protein or its other forms of this invention, cell lines possessing the native receptor protein or its other forms of this invention and cell lines expressing the recombinant receptor protein or its other forms described above are desirable.

For the test compounds, for example, peptides, proteins, non-peptide compounds, synthetic compounds, fermentation products, cell extracts, plant extracts, and animal tissue extracts are used, and these compounds may be novel or publicly known compounds.

The kits for screening compounds or its salts that change the binding property between ligands and the receptor protein or its other forms of this invention contain the receptor protein or its other forms of this invention, cells containing the receptor protein or its other forms of this invention, or the membrane fraction of cells containing the receptor protein or its other forms of this invention.

Examples of the screening kit of this invention are as follow.
1. Reagents for screening
   ① Buffer solution for measurement and washing
      Hanks' balanced salt solution (Gibco Co.) supplemented with 0.05% bovine serum albumin (Sigma Co . ) .
      The solution is sterilized by filtration through a 0.45 µm filter, and stored at 4°C or may be prepared at use.
   ② Standard G-protein-coupled receptor protein
      CHO cells expressing the receptor protein of this invention are bled in 12-well plates at a density of 5 × 10⁵ cells/well and cultured at 37°C under 5% CO₂ and 95% air for two days.
   ③ Labeled ligands
      Aqueous solutions of commercial [³H] -, [¹²⁵I]-, [¹⁴C]-, and [³⁵S]-labeled ligands are stored at 4°C or - 20°C, and diluted to 1 µM with the measurement buffer at use.
   ④ Standard ligand solution
      The ligand is dissolved in and adjusted to 1 mM with PBS containing 0.1% bovine serum albumin (Sigma Co.) and stored at -20°C.
2. Measurement method
   ① CHO cells expressing the receptor protein of this invention are cultured in 12-well culture plates and washed twice with 1 ml of the measurement buffer, and 490 µ1 of the measurement buffer is added to each well.
   ② After adding 5 µl of 10⁻³ - 10⁻¹⁰ M test compound solution, 5 µl of labeled ligand is added, and the cells are incubated at room temperature for one hour. To measure the non-specific binding, 5 µl of 10⁻³ the non-labeled ligand is added in place of the test compound.
   ③ The reaction solution is removed, and the wells are washed three times with 1 ml of the washing buffer. The labeled ligand bound to the cells is dissolved with 0.2N NaOH-1% SDS, and mixed with 4 ml of liquid scintillator A (Wako Pure Chemical Industries, Ltd.)
   ④ The radioactivity is measured using a liquid scintillation counter (Beckman Co.), and the percent maximum binding (PMB) is calculated by the equation below.
      PMB = [(B - NSB)/(B₀ - NSB)] x 100
      PMB: Percent maximum binding
      B : Value obtained in the presence of test compound
      NSB : Non-specific binding
      B₀ : Maximum binding

Compounds or its salts obtained by the screening methods or the screening kits of this invention are compounds that change the binding property between ligands and the receptor protein or its other forms of this invention, and are concretely; (a) compounds that have the G-protein-coupled receptor-mediated cell-stimulating activity (e.g. activity that promotes or inhibits arachidonic acid release, acetylcholine release, intracellular Ca²⁺ release, intracellular cAMP production, intracellular cGMP production, inositol phosphate production, changes in cell membrane potential, phosphorylation of intracellular proteins, activation of c-fos, and pH reduction) (i.e. agonists of the receptor protein of this invention); (b) compounds without the cell stimulating-activity (i.e. antagonists of the receptor protein of this invention); (c) compounds that potentiate the binding affinity between ligands and the G-protein-coupled receptor protein of this invention; or (d) compounds that reduce the binding affinity between ligands and the G-protein-coupled receptor protein of this invention.

The compounds may be peptides, proteins, non-peptide compounds, synthetic compounds, fermentation products, and may be novel or publicly known compounds.

Since agonists of the receptor protein or its other forms of this invention have the same physiological activity as that of ligands for the receptor protein or its other forms of this invention, the agonists are useful as safe and low-toxic pharmaceuticals corresponding to the ligand activity.

Since antagonists of the receptor protein or its other forms of this invention inhibit the physiological activity of ligands for the receptor protein or its other forms of this invention, the antagonists are useful as safe and low-toxic pharmaceuticals that inhibit the ligand activity.

Compounds that potentiate the binding affinity between ligands and the G-protein-coupled receptor protein of this invention are useful as safe and low-toxic pharmaceuticals that potentiate the physiological activity of ligands for the receptor protein and its other forms of this invention.

Compounds that reduce the binding affinity between ligands and the G-protein-coupled receptor protein of this invention are useful as safe and low-toxic pharmaceuticals that decrease the physiological activity of ligands for the receptor protein and its other forms of this invention.

When compounds or its salts obtained by the screening methods or using the screening kits of this invention are used as a component of the pharmaceuticals described above, the compounds can be formulated by the conventional methods. For example, the compounds can be prepared as tablets, capsules, elixir, microcapsules, aseptic solution, or suspension as described for pharmaceuticals containing the receptor protein of this invention.

Since the preparations obtained as described above are safe and low-toxic, they can be administered to, for example, humans and mammals (e.g. rats, rabbits, sheep, pigs, cattle, cats, dogs, monkeys).

The dosage of the compounds or its salts differs depending on the target individual, target organ, symptom, and administration method. When it is administered orally, in general, for schizophrenic patients (60 kg body weight), for example, about 0.1 mg - 100 mg per day, preferably about 1.0 mg - 50 mg per day, more preferably about 1.0 mg - 20 mg per day is administered. When it is administered non-orally, the dosage per dosing differs depending on the target individual, target organ, symptom, and administration method. In case of injection, for schizophrenic patients (60 kg body weight), for example, it is desirable to intravenously inject about 0.01 - 30 mg per day, preferably about 0.1 - 20 mg per day, more preferably about 0.1 - 10 mg per day. Converting the dosage for 60 kg, the protein can be administered to other animals.

### (8) Prophylactic and/or therapeutic drugs for various diseases containing compounds (agonist, antagonist) that change the binding property between ligands and the G-protein-coupled receptor protein of this invention

As described above, the receptor protein of this invention may play some important role in vivo such as a role in the central function. Therefore, compounds (agonist, antagonist) that change the binding property between ligands and the receptor protein of this invention can be used as prophylactic and/or therapeutic drugs for diseases related to dysfunction of the receptor protein of this invention.

When the compounds are used as the prophylactic and/or therapeutic drugs for diseases related to dysfunction of the receptor protein of this invention, the preparations can be obtained according to the conventional methods.

For example, the compounds can be administered orally as sugar coated tablet, capsule, elixiria, and microcapsule, or non-orally as injection such as aseptic solution or suspension in water or other pharmaceutically acceptable liquid. For example, preparations of the compounds can be manufactured by mixing with physiologically acceptable publicly known carrier, flavor, excipient, vehicle, antiseptic, stabilizer, and binder in a unit-dosage form required for generally approved drug preparation. The amount of the active ingredient is set to an appropriate volume within the specified range.

For the additive that may be mixed in tablets and capsules, for example, binders such as gelatin, cornstarch, tragacanth, and acacia, excipients such as crystalline cellulose, imbibers such as cornstarch, gelatin, and alginic acid, lubricants such as magnesium stearate, sweeteners such as sucrose, lactose and saccharin, and flavors such as peppermint, akamono oil and cherry are used. When the dosage form is a capsule, liquid carrier such as fat and oil may be contained. Aseptic compositions for injection can be formulated according to the usual preparation such as dissolving or suspending the active substance in vehicle, e.g. water for injection, and natural plant oils e.g. sesame oil and coconut oil. For the aqueous solution for injection, for example, physiological saline and isotonic solutions (e.g. D-sorbitol, D-mannitol, sodium hydrochloride) containing glucose and other adjuvant are used. Appropriate dissolution-assisting agents, for example, alcohol (e.g. ethanol), polyalcohol (e.g. propylene glycol, polyethylene glycol), nonionic surfactant (e.g. polysorbate 80^{TM}, HCO-50) may be combined. For the oily solution, for example, sesame oil and soybean oil are used, and dissolution-assisting agents such as benzyl benzoate and benzyl alcohol may be combined.

The prophylactic/therapeutic drugs described above may be combined, for example, with buffers (e.g. phosphate buffer solution, sodium acetate buffer solution), analgesics (e.g. benzalkonium chloride, procaine hydrochloride), stabilizers (e.g. human serum albumin, polyethylene glycol), preservatives (e.g. benzylalcohol, phenol), and antioxidants. The preparation for injection is usually filled in appropriate ampoules.

Since the preparations obtained as described above are safe and low toxic, they can be administered to, for example, humans and mammals (e.g. rats, rabbits, sheep, pigs, cattle, cats, dogs, monkeys).

The dosage of the compounds or its salts differs depending on the target individual, target organ, symptom, and administration method. When it is administered orally, in general, for schizophrenic patients (60 kg body weight), for example, about 0.1 mg - 100 mg per day, preferably about 1.0 mg - 50 mg per day, more preferably about 1.0 mg - 20 mg per day is administered. When it is administered non-orally, the dosage per dosing differs depending on the target individual, target organ, symptom, and administration method. In case of injection, for schizophrenic patients (60 kg body weight), for example, it is desirable to intravenously inject about 0.01 - 30 mg per day, preferably about 0.1 - 20 mg per day, more preferably about 0.1 - 10 mg per day. Converting the dosage for 60 kg, the protein can be administered to other animals.

### (9) Quantification of the receptor protein, its partial peptides, and its salts of this invention

Since the antibodies of this invention specifically recognize the receptor protein and its other forms of this invention, the antibodies can be used to quantify the receptor protein and its other forms of this invention in test solutions, especially for quantification by the sandwich immunoassay. This invention provides, for example, the following quantification methods.
(i) Method for quantifying the receptor protein and its other forms of this invention in test solutions in which antibody of this invention, test solution, and labeled receptor protein or its other forms are competitively reacted, and the ratio of the labeled receptor protein or its other forms bound to the antibody is measured.
(ii) Method for quantifying the receptor protein and its other forms of this invention in test solutions in which test solution, antibody of this invention immobilized on carrier, and labeled antibody of this invention are simultaneously or sequentially reacted, and the activity of the label on the immobilized carrier is measured.

In (ii), it is preferable that one antibody recognizes the N-terminal region of the receptor protein or its other forms of this invention, and the other antibody reacts with the C-terminal region of the receptor protein of this invention.

Using monoclonal antibodies against the receptor protein and its other forms of this invention (monoclonal antibodies of this invention), the receptor protein and its other forms of this invention can be measured, and detection by tissue staining can also be performed. For the objective substance, antibody molecules may be used, and F(ab')2, Fab', and Fab fractions of antibody molecule may also be used. Measurement methods using antibodies against the receptor protein and its other forms of this invention are not restricted. Any method can be used in which the amount of antibody, antigen, or antibody-antigen complex corresponding to the amount of antigen (e.g. the amount of the receptor protein) in the test solution is detected by chemical or physical technique and the antigen amount is calculated from a standard curve prepared from standard solutions containing known amount of the antigen. For example, nephrometry, competitive method, immunometric method, and sandwich method are appropriately used, and the sandwich method described below is most preferable in regard to sensitivity and specificity.

For the labeling agent for the methods using labeled substances, for example, radioisotopes, enzymes, fluorescent substances, and luminescent substances are used. For the radioisotope, for example, [¹²⁵I], [¹³¹I], [³H], and [¹⁴C] are used. As the enzyme described above, stable enzymes with high specific activity are preferred, for example, β-galactosidase, β-glucosidase, alkaline phsophatase, peroxidase, and malate dehydrogenase are used. For the fluorescent substance, for example, fluorescamine and fluorescein isothiocyanate are used. For the luminescent substance, for example, luminol, luminol derivatives, luciferin, and lucigenin are used. The biotin-avidin system may be used for binding antibody or antigen to the labeling agent.

For immobilization of antigen or antibody, physical adsorption may be used, and chemical binding methods usually used for insolubilization or immobilization of proteins and enzymes may be used. For the carrier, for example, insoluble polysaccharides such as agarose, dextrin, and cellulose, synthetic resin such as polystyrene, polyacrylamide, and silicon, and glass are used.

In the sandwich method, immobilized monoclonal antibody of this invention is reacted with test solution (primary reaction), then, with labeled monoclonal antibody of this invention (secondary reaction), and the amount of the receptor protein of this invention in the test solution can be quantified by measuring the activity of the label on the immobilizing carrier. The order of the primary and secondary reactions may be reversed, and the reactions may be performed simultaneously or with an interval. The methods for labeling and immobilization can be performed according to the methods described above.

In the immunoassay by the sandwich method, the antibody used for immobilized and labeled antibodies is not necessarily one species, and a mixture of two or more species of antibody may be used to increase the measurement sensitivity.

In the methods for measuring the receptor protein and its other forms of this invention by the sandwich method, for the monoclonal antibodies of this invention used in the primary and secondary reactions, antibodies that bind to different sites of the receptor protein or its other forms are preferred. For the antibodies for the primary and secondary reactions are, for example, when antibody used in the secondary reaction recognizes the C-terminal region of the receptor protein, it is preferable to use antibody recognizing the region other than the C-terminal region for the primary reaction, for example, antibody recognizing the N-terminal region.

Monoclonal antibodies of this invention can be used for measurement systems other than the sandwich method, for example, competitive method, immunometric method, and nephrometry. In the competitive method, antigen in test solution and the labeled antigen are competitively reacted with antibody, and the non-reacted labeled antigen (F) and the labeled antigen bound to the antibody (B) are separated (B/F separation). The amount of the label in B or F is measured, and the amount of the antigen in the test solution is quantified. For the reaction method, liquid phase method using soluble antibody, polyethylene glycol for B/F separation, and the secondary antibody against the soluble antibody, or immobilized method using immobilized antibody as the primary antibody or soluble antibody as the primary antibody and immobilized antibody as the secondary antibody is used.

In the immunometric method, antigen in test solution and immobilized antigen are competitively reacted with a specified amount of labeled antibody, then the immobilized phase and liquid phase are separated, or antigen in test solution and an excess amount of labeled antibody are reacted, then immobilized antigen is added to bind the non-reacted labeled antibody to the immobilized phase, and the immobilized and liquid phases are separated. Then, the amount of the label in either phase is measured to quantify the antigen in the test solution.

In the nephrometry, insoluble precipitate produced after antigen-antibody reaction in gel or solution is quantified. When the amount of antigen in the test solution is small and only a small amount of precipitate is obtained, laser nephrometry using scattering of laser is appropriately used.

For applying these immunological methods to the measurement methods for this invention, no specific conditions or procedures are necessary. Systems for measuring the receptor protein and its salts of this invention are constructed by adding the usual technical consideration in the art to the conventional conditions and procedures. The details of these general technical means can be referred to reviews and texts. [For example, Irie, H. ed. 'Radioimmunoassay' (Kodansha, 1974), Irie, H. ed. 'Sequel to the Radioimmunoassay' (Kodansha, 1979), Ishikawa, E. et al. ed. 'Immunoenzyme assay' (Igakushoin, 1978), Ishikawa, E. et al. ed. 'Immunoenzyme assay' (2nd ed.) (Igakushoin, 1982), Ishikawa, E. et al. ed. 'Immunoenzyme assay' (3rd ed.) (Igakushoin, 1987), Methods in ENZYMOLOGY Vol. 70 (Immunochemical Techniques (Part A)), Vol. 73 (Immunochemical Techniques (Part B)), Vol. 74 (Immunochemical Techniques (Part C)), Vol. 84 (Immunochemical Techniques (Part D: Selected Immunoassays)), Vol. 92 (Immunochemical Techniques (Part E: Monoclonal Antibodies and General Immunoassay Methods)), Vol. 121 (Immunochemical Techniques (Part I: Hybridoma Technology and Monoclonal Antibodies)) (Academic Press Publishing).

Thus, using the antibodies of this invention, the receptor protein and its salts of this invention can be quantified with high sensitivity.

Quantifying the receptor protein or its salts of this invention in vivo using the antibodies of this invention, various diseases related to dysfunction of the receptor protein of this invention can be diagnosed.

The antibodies of this invention can also be used for specific detection of the receptor protein and its other forms of this invention present in test samples such as body fluids and tissues. The antibodies are also used for preparation of antibody columns for purification of the receptor protein and its other forms of this invention, detection of the receptor protein and its other forms of this invention in each fraction in purification, and analysis of fate of the receptor protein of this invention in the test cells.

### (10) Methods for screening compounds that change the amount of the receptor protein or its partial peptides of this invention in cell membranes

Since the antibodies of this invention specifically recognize the receptor protein, its partial peptides, or salts of this invention, the antibodies can be used for screening of compounds that change the amount of the receptor protein or its partial peptides of this invention in cell membranes.

For example, this invention provides the following methods.
(i) Methods for screening compounds that change the amount of the receptor protein or its partial peptides in cell membranes in which ① blood, ② specified organs, ③ tissues or cells isolated from the organs from non-human mammals are disrupted, and the cell membrane fraction is separated, then, the receptor protein or its partial peptides of this invention contained in the cell membrane fraction are quantified.
(ii) Method for screening compounds that change the amount of the receptor protein or its partial peptides of this invention in cell membranes in which transformants expressing the receptor protein or its partial peptides of this invention are disrupted and the membrane fraction is separated, and then, the receptor protein or its partial peptides of this invention contained in the cell membrane fraction are quantified.
(iii) Method for screening compounds that change the amount of the receptor protein or its partial peptides in cell membranes in which ① blood, ② specified organs, ③ tissues or cells isolated from the organs from non-human mammals are sectioned, then, the protein on the cell membrane is confirmed by quantifying the staining intensity of the receptor protein in the cell surface layer using immunostaining.
(iv) Method for screening compounds that change the amount of the receptor protein or its partial peptides in cell membranes in which transformants expressing the receptor protein or its partial peptides of this invention are sectioned, then the protein on the cell membrane is confirmed by quantifying the staining intensity of the receptor protein in the cell surface layer using immunostaining.

Concretely, the receptor protein and its partial peptides of this invention contained in cell membrane fraction are quantified as follows.
(i) Normal or disease models of non-human mammals (e.g. mice, rats, rabbits, sheep, pigs, cattle, cats, dogs, monkeys, more concretely, dementia rats, obese mice, arteriosclerotic rabbits, tumor-bearing mice) receive administration of a drug (e.g. nootroopic drugs, hypotensive drugs, anticancer drugs, antiobesitic drugs) or physical stress (e.g. soaking stress, electric shock, light and darkness, low temperature), and the blood, specific organ (e.g. brain, liver, kidneys), or tissue or cells isolated from the organs are obtained after a specified time. The obtained organ, tissue, or cells are suspended in, for example, appropriate buffer solution (e.g. Tris hydrochloride buffer, phosphate buffer, Hepes buffer), and the organ, tissue, or cells are disrupted, and the cell membrane fraction is obtained using surfactants (e.g. Triton-X 100^{TM}, Tween 20^{TM}) and techniques such as centrifugation, filtration, and column fractionation.
   The cell membrane fraction is a fraction containing the abundant cell membrane obtained after disruption of the cells by publicly known methods. The cell disruption methods include crushing the cells using a Potter-Elvehjem homogenizer, disruption using a Waring blender or polytron (Kinematica Co.), disruption by ultrasonication, and disruption by passing the cells through a narrow nozzle with compressing the cells using a French Press. For the cell membrane fractionation method, fractionation based on centrifugal force such as centrifugation for fractionation and density gradient centrifugation are mainly used. For example, disrupted cell suspension is centrifuged at a low speed (500 - 3,000 rpm) for a short time (usually about 1 - 10 minutes), then the supernatant is centrifuged at a high speed (15,000 - 30,000 rpm) for usually 30 minutes - 2 hours, and the obtained precipitate is used as the membrane fraction. The membrane fraction contains many membrane components such as the expressed receptor protein or its other forms, and phospholipids and membrane proteins derived from the cells.
   The receptor protein or its partial peptides of this invention contained in the cell membrane fraction can be quantified by, for example, sandwich immunoassay and western blot analysis using the antibodies of this invention.
   The sandwich immunoassay can be performed as described above, and the western blot can be performed by publicly known methods.
(ii) Transformants expressing the receptor protein or its partial peptides of this invention are prepared according to the method described above, and the receptor protein or its partial peptides of this invention contained in the cell membrane fraction can be quantified.

Compounds that change the amount of the receptor protein or its partial peptides of this invention in cell membranes can be screened as follows.
(i) To normal or disease models of non-human mammals, test compound is administered at a specified time before (30 minutes - 24 hours before, preferably 30 minutes - 12 hours before, more preferably 1 hour - 6 hours before), at a specified time after (30 minutes - 3 days after, preferably 1 hour - 2 days after, more preferably 1 hour - 24 hours after), or simultaneously with a drug or physical stress. At a specified time (30 minutes - 3 days, preferably 1 hour - 2 days, more preferably 1 hour - 24 hours) after administration of the test compound, the receptor protein or its partial peptides of this invention in the cell membrane can be quantified.
(ii) When transformants are cultured by the conventional method, test compound is mixed in the culture medium. After culture for a specified time (after 1 day - 7 days, preferably after 1 day - 3 days, more preferably after 2 days - 3 days), the receptor protein or its partial peptides of this invention in the cell membrane can be quantified.
   Concretely, the receptor protein and its partial peptides of this invention in cell membrane fractions are confirmed as follows.
(iii) Normal or disease models of non-human mammals (e.g. mice, rats, rabbits, sheep, pigs, cattle, cats, dogs, monkeys, more concretely, dementia rats, obese mice, arteriosclerotic rabbits, tumor-bearing mice) receive administration of a drug (e.g. nootroopic drugs, hypotensive drugs, anticancer drugs, antiobesitic drugs) or physical stress (e.g. soaking stress, electric shock, light and darkness, low temperature), and the blood, specific organ (e.g. brain, liver, kidneys), or tissue or cells isolated from the organ are obtained after a specified time. Tissue sections are prepared from the obtained organ, tissue, or cells according to the conventional method, and immunostained with the antibody of this invention. Quantifying the staining intensity of the receptor protein in the cell surface layer, the protein on the cell membrane can be confirmed, and the amount of the receptor protein or its partial peptides of this invention can be quantitatively or qualitatively confirmed.
(iv) The receptor protein or its partial peptides of this invention can also be confirmed by the similar method using transnformants expressing the receptor protein or its partial peptides of this invention.

Compounds or its salts obtained by the screening methods for this invention are compounds that change the amount of the receptor protein or its partial peptides of this invention, and are concretely; (a) compounds that potentiate the cell-stimulating activity mediated by the G-protein-coupled receptor (e.g. activity that promotes or inhibits arachidonic acid release, acetylcholine release, intracellular Ca²⁺ release, intracellular cAMP production, intracellular cGMP production, inositol phosphate production, changes in cell membrane potential, phosphorylation of intracellular proteins, activation of c-fos, and pH reduction) by increasing the amount of the receptor protein or its partial peptides of this invention in cell membranes; and (b) compounds that reduce the cell stimulating activity by decreasing the amount of the receptor protein or its partial peptides of this invention in cell membranes.

The compounds include peptides, proteins, non-peptide compounds, synthetic compounds, and fermentation products, and may be novel or publicly known compounds.

The compounds that potentiate the cell-stimulating activity are useful as safe and low-toxic pharmaceuticals for potentiation of the physiological activity of the receptor protein and its other forms of this invention.

The compounds that decrease the cell-stimulating activity are useful as safe and low-toxic pharmaceuticals for reduction of the physiological activity of the receptor protein and its other forms of this invention.

When compounds or its salts obtained by the screening methods for this invention are used as a pharmaceutical component, preparations can be prepared according to the conventional methods. For example, as described above for preparation of the pharmaceuticals containing the receptor protein of this invention, the compounds can be prepared as tablets, capsules, elixiria, microcapsules, aseptic solution, and suspension.

Since the preparations obtained as described above are safe and low-toxic, they can be administered to humans and mammals (e.g. rats, rabbits, sheep, pigs, cattle, cats, dogs, monkeys).

The dosage of the compounds or its salts differs depending on the target individual, target organ, symptom, and administration method. When it is administered orally, in general, for schizophrenic patients (60 kg body weight), about 0.1 mg - 100 mg per day, preferably about 1.0 mg - 50 mg per day, more preferably about 1.0 mg - 20 mg per day is administered. When it is administered non-orally, the dosage per dosing differs depending on the target individual, target organ, symptom, and administration method. In case of injection, for schizophrenic patients (60 kg body weight), for example, usually it is desirable to intravenously inject about 0.01 - 30 mg per day, preferably about 0.1 - 20 mg per day, more preferably about 0.1 - 10 mg per day. Converting the dosage for 60 kg, the protein can be administered to other animals.

### (11) Prophylactic and/or therapeutic drugs for various diseases containing compounds that change the amount of the receptor protein or its partial peptides of this invention in cell membrane

As described above, the receptor protein of this invention is considered to play some important role such as a role in the central function. Therefore, compounds that change the amount of the receptor protein or its partial peptides of this invention in cell membrane can be used as prophylactic and/or therapeutic drugs for diseases related to dysfunction of the receptor protein of this invention.

When the compounds are used as prophylactic and/or therapeutic drugs for diseases related to dysfunction of the receptor protein of this invention, the preparations can be obtained according to the conventional methods.

For example, the compounds can be administered orally as a sugar coated tablet, capsule, elixir, and microcapsule, or non-orally as injection such as aseptic solution and suspension in water or other pharmaceutically acceptable liquid. For example, preparations of the compounds can be manufactured by mixing with physiologically acceptable publicly known carrier, flavor, excipient, vehicle, antiseptic, stabilizer, and binder in a unit-dosage form required for generally approved drug preparation. The amount of the efficient ingredient is set to an appropriate volume within the specified range.

For the additive that may be mixed in tablets and capsules, for example, binders such as gelatin, cornstarch, tragacanth, and acacia, excipients such as crystalline cellulose, imbibers such as cornstarch, gelatin, and alginic acid, lubricants such as magnesium stearate, sweeteners such as sucrose, lactose and saccharin, and flavors such as peppermint, akamono oil and cherry are used. When the dosage form is a capsule, liquid carrier such as fat and oil may be contained. Aseptic compositions for injection can be formulated according to the usual preparation such as dissolving or suspending the active substance in vehicle, e.g. water for injection, and natural plant oils e.g. sesame oil and coconut oil. For the aqueous solution for injection, for example, physiological saline and isotonic solutions (e.g. D-sorbitol, D-mannitol, sodium hydrochloride) containing glucose and other adjuvant are used. Appropriate dissolution-assisting agents, for example, alcohol (e.g. ethanol), polyalcohol (e.g. propylene glycol, polyethylene glycol), nonionic surfactant (e.g. polysorbate 80^{TM}, HCO-50) may be combined. For the oily solution, for example, sesame oil and soybean oil are used, and dissolution-assisting agents such as benzyl benzoate and benzyl alcohol may be combined.

The prophylactic/therapeutic drugs described above may be combined with buffers (e.g. phosphate buffer solution, sodium acetate buffer solution), analgesics (e.g. benzalkonium chloride, procaine hydrochloride), stabilizers (e.g. human serum albumin, polyethylene glycol), preservatives (e.g. benzylalcohol, phenol), and antioxidants. The preparation for injection is usually filled in appropriate ampoules.

Since the preparations obtained as described above are safe and low toxic, they can be administered to, for example, humans and mammals (e.g. rats, rabbits, sheep, pigs, cattle, cats, dogs, monkeys).

The dosage of the receptor protein and its other forms of this invention differs depending on the target individual, target organ, symptom, and administration method. When it is administered orally, in general, for schizophrenic patients (60 kg body weight), for example, about 0.1 mg - 100 mg per day, preferably about 1.0 mg - 50 mg per day, more preferably about 1.0 mg - 20 mg per day is administered. When it is administered non-orally, the dosage per dosing differs depending on the target individual, target organ, symptom, and administration method. In case of injection, for schizophrenic patients (60 kg body weight), for example, it is desirable to intravenously inject about 0.01 - 30 mg per day, preferably about 0.1 - 20 mg per day, more preferably about 0.1 - 10 mg per day. Converting the dosage for 60 kg, the protein can be administered to other animals.

### (12) Neutralization with antibodies against the receptor protein, its partial peptides, and its salts of this invention

The neutralizing activity of antibodies against the receptor protein, its partial peptides, and its salts of this invention means the activity of inactivating the signal transduction function involving the receptor protein. Therefore, when the antibody has neutralizing activity, the signal transduction involving the receptor protein, for example, the cell-stimulating activity mediated via the receptor protein (e.g. activity that promotes or inhibits arachidonic acid release, acetylcholine release, intracellular Ca²⁺ release, intracellular cAMP production, intracellular cGMP production, inositol phosphate production, changes in cell membrane potential, phosphorylation of intracellular proteins, activation of c-fos, and pH reduction) can be inactivated. Thus, the antibody can be used for prophylaxis and/or therapy of diseases caused by overexpression of the receptor protein.

### (13) Preparation of non-human animals containing the DNA encoding the G-protein-coupled receptor protein of this invention

Using the DNA of this invention, non-human transgenic animals expressing the receptor protein or its other forms can be prepared. For the non-human animals, mammals (e.g. rats, mice, rabbits, sheep, pigs, cattle, cats, dogs, monkeys) (abbreviated as animals hereinafter) can be used, and mice and rabbits are particularly appropriate.

To introduce the DNA of this invention into target animals, it is generally advantageous to use the DNA as a gene construct ligated to downstream of a promoter that can express the DNA in animal cells. For example, when the rabbit-derived DNA of this invention is introduced, the gene construct, in which the DNA is ligated to downstream of a promoter that can expresses DNA of this invention derived from animals containing highly homologous DNA of this invention, is microinjected to rabbit fertilized egg, and the DNA-introduced animal producing a high level of the receptor protein or its other forms of this invention can be prepared. For the promoter, for example, virus-derived promoters and ubiquitous expression promoters such as metallothionein promoter may be used, but preferably, promoters of NGF gene and enolase that are specifically expressed in the brain are used.

The DNA transfer of the DNA of this invention at the fertilized egg cell stage secures the presence of DNA in all germ and somatic cells in the prepared animal. The presence of the receptor protein or its other forms of this invention in the germ cells in the DNA-transferred animal means that all germ and somatic cells contain the receptor protein or its other forms of this invention in all progenies of the animal. The progenies of the animal that took over the gene contain the receptor protein or its other forms of this invention in all germ and somatic cells.

The DNA-transferred animals of this invention can be bled in the conventional environment as animals carrying the DNA after confirming the stable retention of the gene in the animals by mating. Furthermore, by mating male and female animals containing the objective DNA, homozygote animals having the transferred gene on both homologous chromosomes can be obtained, and by mating the male and female homozygotes, bleeding in which all progenies contain the DNA can be performed.

Since the receptor protein or its other forms are highly expressed in the DNA-transferred animals of this invention, the animals are useful for screening of agonists or antagonists of the receptor protein and its other forms of this invention.

The DNA-transferred animals of this invention can also be used as cell sources for tissue culture. The receptor protein and its other form of this invention can be analyzed by, for example, directly analyzing the DNA or RNA in tissues of the DNA-transferred mice of this invention, or by analyzing tissues containing the receptor protein expressed from the gene. Cells from tissues containing the receptor protein or its other forms of this invention are cultured by the standard tissue culture technique. Using these cells, for example, the function of the cells of tissues that are generally difficult to culture such as cells derived from the brain and peripheral tissues can be studied. Using these cells, for example, it is possible to select pharmaceuticals that increase the function of various tissues. If a highly protein expressing cell line is available, the receptor protein or its other forms of this invention can be purified from the cell line.

In this specification and drawings, the codes of bases and amino acids are according to IUPAC-IUB Commission on Biochemical Nomenclature or common codes in the art. The examples are shown below. For amino acids that may have the optical isomer, L form is presented unless it is specified.
DNA : deoxyribonucleic acid
cDNA : complementary deoxyribonucleic acid
A : adenine
T : thymine
G : guanine
C : cytosine
RNA : ribonucleic acid
mRNA : messenger ribonucleic acid
dATP : deoxyadenosine triphosphate
dTTP : deoxythymidine triphosphate
dGTP : deoxyguanosine triphosphate
dCTP : deoxycytidine triphosphate
ATP : adenosine triphosphate
EDTA : ethylenediaminetetraacetic acid
SDS : sodium dodecyl sulfate
Gly : glycine
Ala : alanine
Val : valine
Leu : leucine
Ile : isoleucine
Ser : serine
Thr : threonine
Cys : cysteine
Met : methionine
Glu : glutamic acid
Asp : aspartic acid
Lys : lysine
Arg : arginine
His histidine
Phe : phenylalanine
Tyr : tyrosine
Trp : tryptophan
Pro : proline
Asn : aspargine
Gln : glutamine
pGlu : pyroglutamic acid
Me : methyl group
Et : ethyl group
Bu : butyl group
Ph : phenyl group
TC : thiazolidine-4(R)-carboxyamide group

Substituents, protecting groups, and reagents generally used in this specification are presented as the codes below.
Tos : p-toluenesulfonyl
CHO : formyl
Bzl benzyl
Cl₂Bzl : 2,6-dichlorobenzyl
Bom benzyloxymethyl
Z benzyloxycarbonyl
Cl-Z : 2-chlorobenzyl oxycarbonyl
Br-Z : 2-bromobenzyl oxycarbonyl
Boc : t-butoxycarbonyl
DNP dinitrophenol
Trt : trityl
Bum : t-butoxymethyl
Fmoc : N-9-fluorenyl methoxycarbonyl
HOBt : 1-hydroxybenztriazole
HOOBt : 3,4-dihydro-3-hydroxy-4-oxo-1,2,3-benzotriazine
HONB : 1-hydroxy-5-norbornene-2,3-dicarboxyimide
DCC : N,N'-dichlorohexylcarbodiimide

The SEQ IDs in the sequence listing of this specification present the following sequences.

### [SEQ ID:1]

The amino acid sequence of the rat cerebellum-derived novel G-protein-coupled receptor protein rCB7T084 of this invention

### [SEQ ID:2]

The base sequence of cDNA encoding the rat cerebellum-derived novel G-protein-coupled receptor protein rCB7T084 of this invention having the amino acid sequence shown in SEQ ID:1.

### [SEQ ID:3]

The base sequence of primer 1 used for cloning the cDNA encoding the rat cerebellum-derived novel G-protein-coupled receptor protein rCB7T084 of this invention.

### [SEQ ID:4]

The base sequence of primer 2 used for cloning the cDNA encoding the rat cerebellum-derived novel G-protein-coupled receptor protein rCB7T084 of this invention.

*Escherichia coli* transformant DH10B/pAK-rCB084 obtained in the example 1 described below was deposited with the Ministry of International Trade and Industry, Agency of Industrial Science and Technology, National Institute of Bioscience and Human Technology (NIBH) as deposit number FERM BP-6485 on September 4, 1998 and with Institute for Fermentation, Osaka (IFO) as deposit number IFO 16199 on August 17, 1998.

The present invention is explained in detail below by showing examples, but the description is not limited the scope of this invention. The genetic engineering procedures using *Escherichia coli* were performed according to the methods described in the Molecular Cloning.

### Example 1 Cloning and sequencing of cDNA encoding the rat cerebellum-derived G-protein-coupled receptor protein

Using rat cerebellum cDNA as the template and two primers: primer 1 (SEQ ID:3) and primer 2 (SEQ ID:4), PCR reaction was performed. The compositions of the reaction solution were 1/10 volume of the cDNA for the template, 1/50 volume of Advantage cDNA Polymerase Mix (CLONTEC Inc.), 0.2 µM primer 1 (SEQ ID:3), 0.2 µM primer 2 (SEQ ID:4), 200 µM dNTPs, and the buffer attached to the enzyme, making the final volume of 25 µl. In the PCR reaction; ① the reaction solution was heated at 95°C for 30 seconds; ② a cycle consisting of heating at 94°C for 5 seconds followed by 70°C for 5 minutes was repeated five times; ③ a cycle of heating at 94°C for 5 seconds followed by 68°C for 5 minutes was repeated five times ; ④ a cycle of 94°C for 5 seconds followed by 65°C for 5 minutes was repeated 35 times; and ⑤ at last, elongation reaction was performed at 65°C for 5 minutes. The PCR products were subcloned into plasmid vector pCRII (Invitrogen Inc.) according to the instruction attached to TA cloning kit (Invitrogen Inc.). The vectors were introduced in *Escherichia coli* DH5α, and clones containing the cDNA were selected on LB agar plates containing ampicillin. The sequence of each clone was analyzed, and the cDNA (SEQ ID:2) encoding the novel G-protein-coupled receptor protein was obtained. The novel G-protein-coupled receptor protein containing the amino acid sequence (SEQ ID:1) deduced from the cDNA was designated as rCB7T084.

Plasmid pAK-rCB084 in which the cDNA (SEQ ID:2) encoding the rat cerebellum-derived G-protein-coupled receptor protein rCB7T084 of this invention was subcloned was introduced in *Escherichia coli* DH10B by the publicly known method, and the transformant *Escherichia* coli DH10B/pAK-rCB084 was obtained.

Plasmid pCRII-rCB7T084 in which the cDNA (SEQ ID:2) encoding the rat cerebellum-derived G-protein-coupled receptor protein rCB7T084 of this invention was subcloned was introduced in *Escherichia* coli DH5α by the publicly known method, and the transformant *Escherichia* coli DH5α/pCRII-rCB7T084 was obtained. Example 2 Preparation of rCB7T084-expressing CHO cells

After the transformant E. *coli* DH5α/pCRII-rCB7T084 prepared in Example 1 was cultured, the plasmid DNA of pCRII-rCB7T084 was prepared using Plasmid Midi kit (Qiagen Inc.). The cDNA encoding the G-protein-coupled receptor protein rCB7T084 of this invention obtained from this plasmid was cloned into plasmid vector pcDNA3.1/V5/His to construct the protein expression vector pcDNA3.1-rCB084. A large amount of plasmid DNA was prepared from the obtained plasmid using Plasmid Midi kit (Qiagen Inc.), and introduced into CHO dhfr⁻ cells using Cellfect transfection kit (Amersham Pharmacia Biotech Inc.) according to the protocol attached to the kit. Ten mg of DNA was co-precipitated with calcium phosphate in suspension, and added to a 10 cm dish in which 5 x 10⁵ or 1 x 10⁶ CHO dhfr cells were seeded before 24 hours, and the cells were cultured in MEMα containing 10% fetal calf serum for one day. After passage, the cells were cultured in selection medium MEMα containing 0.4 mg/ml G418 (Gibco BRL Inc.) and 10% dialyzed fetal calf serum. The colonies of transformed cells (CHO/rCB084) growing in the selection medium were selected as rCB7T084-expressing CHO cells.

Total RNA was extracted from the selected rCB7T084-expressing CHO cells by the conventional method, and the amount and the copy numbers of rCB7T084 mRNA were estimated by the TaqMan method. The results are shown in the Table below.

| Clone No. | Expression level (copies/ng total RNA) | |
|---|---|---|
| | 1st measurement | 2nd measurement |
| 3 | 18666 | 17486 |
| | 18278 | 24369 |
| 4 | 181736 | 190185 |
| | 159347 | 175345 |
| 17 | 50429 | 34503 |
| | 44990 | 41239 |

### INDUSTRIAL APPLICABILITY

The G-protein-coupled receptor protein, its partial peptides, and its salts of this invention and the polynucleotides encoding thereof (e.g. DNA, RNA, and its derivatives) can be used for; ① determination of ligands (agonist); ② preparation of antibody and antiserum; ③ construction of expression systems for recombinant receptor protein; ④ development of receptor binding assay systems using the expression systems and screening of pharmaceutical candidate compounds; ⑤ execution of drug design based on comparison with structurally similar ligand receptors; ⑥ reagents for preparation of probes and PCR primers for gene diagnosis; ⑦ preparation of transgenic animals; and ⑧ pharmaceuticals such as gene prophylactic/therapeutic drugs.

## Claims

1. A G-protein-coupled receptor protein and a salt form of said protein characterized by containing an amino acid sequence that is identical or substantially identical with the amino acid sequence presented in SEQ ID:1.

2. A partial peptide of the G-protein-coupled receptor protein described in claim 1 and a salt form of said peptide.

3. A polynucleotide containing a polynucleotide containing a base sequence encoding the G-protein-coupled receptor protein described in claim 1.

4. A polynucleotide according to claim 3 wherein said polynucleotide is DNA.

5. A polynucleotide according to claim 3 wherein said polynucleotide contains the base sequence presented in SEQ ID:2.

6. A recombinant vector containing a polynucleotide described in claim 3.

7. A transformant transformed by a recombinant vector described in claim 6.

8. A method for manufacturing the G-protein-coupled receptor protein or a salt form of said protein, wherein a transformant described in claim 7 is cultured and the G-protein-coupled receptor protein described in claim 1 is produced.

9. An antibody against the G-protein-coupled receptor protein described in claim 1, a fragment peptide described in claim 2, or a salt form of said protein or peptide.

10. A neutralizing antibody according to claim 9, wherein said antibody inactivates signal transduction of the G-protein-coupled receptor protein described in claim 1.

11. A diagnostic drug that contains an antibody described in claim 9.

12. A ligand for the G-protein-coupled receptor protein or a salt form of said protein described in claim 1, wherein said ligand is obtained using the G-protein-coupled receptor protein described in claim 1, a fragment peptide described in claim 2, or a salt form of said protein or peptide.

13. A pharmaceutical containing a ligand for the G-protein-coupled receptor protein described in claim 12.

14. A method for determining a ligand for the G-protein-coupled receptor protein or a salt form of said protein described in claim 1, wherein the G-protein-coupled receptor protein described in claim 1, a fragment peptide described in claim 2, or a salt form of said protein or peptide is used.

15. A method for screening a compound that changes the binding property between a ligand and the G-protein-coupled receptor protein or a salt form of said protein described in claim 1, wherein the G-protein-coupled receptor protein described in claim 1, a fragment peptide described in claim 2, or a salt form of said protein or peptide is used.

16. A kit for screening a compound that changes the binding property between a ligand and the G-protein-coupled receptor protein or a salt form of said protein described in claim 1, wherein the G-protein-coupled receptor protein described in claim 1, a fragment peptide described in claim 2, or a salt form of said protein or peptide is contained.

17. A compound and a salt form of said compound that change the binding property between a ligand and the G-protein-coupled receptor protein or a salt form of said protein described in claim 1 obtained using a screening method described in claim 15 or a screening kit described in claim 16.

18. A pharmaceutical containing a compound or a salt form of said compound that changes the binding property between a ligand and the G-protein-coupled receptor protein or a salt form of said protein described in claim 1 obtained using a screening method described in claim 15 or a screening kit described in claim 16.

19. A polynucleotide that hybridizes to a polynucleotide described in claim 3 under a high stringent condition.

20. A polynucleotide containing a base sequence complementary to a polynucleotide described in claim 3 or a part of said base sequence.

21. A method for quantifying mRNA of the G-protein-coupled receptor protein described in claim 1, wherein a polynucleotide described in claim 3 or a part of said polynucleotide is used.

22. A method for quantifying the G-protein-coupled receptor protein described in claim 1, wherein an antibody described in claim 9 is used.

23. A diagnostic method for a disease related to function of the G-protein-coupled receptor protein described in claim 1, wherein a quantification method described in claim 21 or 22 is used.

24. A method for screening a compound or a salt form of said compound that changes the expression level of the G-protein-coupled receptor protein described in claim 1, wherein a quantification described in claim 21 is used.

25. A method for screening a compound that changes the amount of the G-protein-coupled receptor protein or a salt form of said protein described in claim 1 in cell membrane, wherein a quantification method described in claim 22 is used.

26. A compound and a salt form of said compound that change the expression level of the G-protein-coupled receptor protein described in claim 1 obtained using a screening method described in claim 24.

27. A compound and a salt form of said compound that change the amount of the G-protein-coupled receptor protein described in claim 1 in cell membrane obtained using a screening method described in claim 25.
